# EUROPEAN PATENT SPECIFICATION

(11) **EP 2 203 180 B1**
(45) Date of publication and mention of the grant of the patent: **21.11.2012**
(21) Application number: 08840928.9
(22) Date of filing: 21.10.2008
(51) Int. Cl.: A61K 38/21, A61P 31/12, C07K 14/565, C07K 16/00, C07K 19/00

(54) **SINGLE IFN-BETA FUSED TO A MUTATED IGG FC FRAGMENT**
MIT MUTIERTEM IGG-FC-FRAGMENT ANELLIERTES EINZEL-IFN-BETA
IFN-BÊTA UNIQUE FUSIONNÉ À UN FRAGMENT FC D'IGG MUTÉ

(30) Priority: 22.10.2007 EP 07118980; 11.12.2007 US 7142 P
(43) Date of publication of application: 07.07.2010
(73) Proprietor: Merck Serono S.A., 1267 Coinsins (CH)
(72) Inventor: HJELMSTROM, Peter, S-75312 Uppsala (SE); IMHOF, Markus, CH-1071 Chexbres (CH); FEGER, George, F-01710 Thoiry (FR); JENKINS, Nigel, Dublin 9 (IE); DUPRAZ, Philippe, CH-1023 Crissier (CH); POWER, Christine, F-01710 Thoiry (FR); MAGNENAT, Laurent, CH-1202 Genève (CH); CHATELLARD, Philippe, CH-1005 Lausanne (CH); PANKIEWICZ, Renata, 1092 Belmont (CH); DECHAVANNE, Vincent, F-74160 Beaumont (FR); BOZZATO, Gian Battista, deceased (IT)
(74) Representative: Merck Serono SA - Geneva Intellectual Property
(86) International application number: PCT/EP2008/064224
(87) International publication number: WO 2009/053368

(56) References cited:
- WO-A-2004/004798
- WO-A-2006/000448
- WO-A-2006/074199
- WO-A-2006/077266
- WO-A-2006/127757
- US-A1- 2003 082 749
- BITONTI ET AL: "Pulmonary administration of therapeutic proteins using an immunoglobulin transport pathway" ADVANCED DRUG DELIVERY REVIEWS, AMSTERDAM, NL, vol. 58, no. 9-10, 11 November 2006 (2006-11-11), pages 1106-1118, XP005761773 ISSN: 0169-409X
- DUMONT JENNIFER A ET AL: "MONOMERIC FC FUSIONS: IMPACT ON PHARMACOKINETIC AND BIOLOGICAL ACTIVITY OF PROTEIN THERAPEUTICS" BIODRUGS, AUCKLAND, NZ, vol. 20, no. 3, 2006, pages 151-160, XP009082835 ISSN: 1173-8804
- ETTINGER R ET AL: "Disrupted splenic architecture, but normal lymph node development in mice expressing a soluble lymphotoxin-beta receptor-IgG1 fusion protein." PROCEEDINGS OF THE NATIONAL ACADEMY OF SCIENCES OF THE UNITED STATES OF AMERICA 12 NOV 1996, vol. 93, no. 23, 12 November 1996 (1996-11-12), pages 13102-13107, XP002470115 ISSN: 0027-8424

## Description

### Field of the invention

The present invention relates to a single IFN-beta fused to a mutated IgG Fc fragment (hereafter referred to as sIFNbeta-IgGFcmut), nucleotides encoding the same, vectors and expression vectors comprising the same, host cells transformed therewith and their use as medicament as well as their use in particular to treat multiple sclerosis. The single IFN-beta fused to a mutated IgG Fc fragment comprises two subunits, the first subunit comprising a mutated IgG Fc arm not linked to a IFN-beta protein and the second subunit comprising a mutated IgG Fc arm linked to a single IFN-beta protein.

### Background of the invention

It has been reported (cf Spiekermann GM *et al.* 2002) that proteins comprising the Fc fragment of an immunoglobulin (Ig) linked to a protein of interest, e.g. Erythropoietin (EPO), allow for inhalable administration, apparently through binding to the MHC class I-related Fc-receptor, FcRn, present in adult human lungs that recognizes, binds and takes up the Fc fragment of immunoglobulins. Fusing a protein to an immunoglobulin Fc fragment allows the hybrid protein to be recognized by the FcRn. The immunoglobulin Fc fragment consists of two identical arms which comprise the hinge region (H) and the second (CH₂) and third (CH₃) domain of an antibody heavy chain.

FcRn is active in adult (human but not rodent) epithelial tissue and expressed in the lumen of the intestines, pulmonary airways, nasal surfaces, vaginal surfaces, colon and rectal surfaces (US 6,485,726). Proteins comprising an FcRn binding partners (e.g. IgG, Fusion proteins containing Fc fragments) can be effectively shuttled across epithelial barriers by FcRn, thus providing a non-invasive means to systemically administer a desired therapeutic molecule.

It is know that the Fc fragment, also called Fc domain, of immunoglobulins (e.g. IgGs) is responsible for the so called effector functions associated with the administration of an immunoglobulin (IgG) or of a fusion protein comprising said Fc domain (IgGFc). Such effector functions include antibody-dependent cellular cytotoxicity (ADCC) through interactions with Fc receptors (FcγRs) and complement-dependent cytotoxicity (CDC) by binding to the complement component 1q (C1q). IgG isoforms exert different levels of effector functions. Human IgG1 and IgG3 have strong ADCC and CDC effects while human IgG2 exerts weak ADCC and CDC effects. Human IgG4 displays weak ADCC and no CDC effects.

In ADCC, the Fc region of an antibody (e.g. IgG) binds to Fc receptors (FcγRs) on the surface of immune effector cells such as natural killers and macrophages, leading to the phagocytosis or lysis of the targeted cells.

In CDC, the antibody (e.g. IgG) kills the targeted cells by triggering the complement cascade at the cell surface. IgG isoforms exert different levels of effector functions increasing in the order of IgG4 < IgG2 < IgG1 ≤ IgG3.

Interferons are naturally secreted by infected cells and were first identified in 1957. Their name is derived from the fact that they "interfere" with viral replication and production. Interferon beta is a well-known interferon that is currently used to treat multiple sclerosis.

It has been reported (WO 2005/001025) that monomer-dimer hybrids, with one biologically active molecule, but two portions of an immunoglobulin constant region, e.g. two FcRn binding partners, function and can be transported more effectively than homodimers, also referred to herein simply as dimers or higher order multimers with two or more copies of the biologically active molecule. Monomer-dimer hybrids of interferon beta are disclosed in WO 2005/001025. A monomer-dimer hybrid comprising human interferon beta fured to the human IgG1 FC region is disclosed in WO 2006/074 199.

### Summary of the invention

In a first aspect, the invention provides a single IFN-beta fused to a mutated IgG Fc domain (hereafter referred to as sIFNbeta-IgGFcmut). The sIFNbeta-IgGFcmut' of the invention consists of two subunits, the first subunit comprises a mutated IgG Fc arm not linked (Fcmut arm) to a IFN-beta protein and the second subunit comprises a mutated IgG Fc arm linked to a single IFN-beta protein (IFNbeta-Fcmut arm) wherein said first subunit consists of SEQ ID NO: 3 and said second subunit consists of SEQ ID NO: 8. The mutations introduced result in a lower ADCC and in a lower CDC. In a preferred embodiment the mutations are L234A, L235E, G237A, A330S and P331S. In another aspect, the invention provides a polynucleotide, e.g. a DNA, encoding a sIFNbeta-IgGFcmut according to the present invention, vectors and more particularly expression vectors containing said DNA.

In another aspect, the invention provides a host cell, preferably a CHO cell, containing, e.g. as a result of a transfection, a vector and in particular an expression vector according to the invention.

In another aspect, the invention provides a pharmaceutical composition comprising a sIFNbeta-IgGFcmut according to the present invention and its use as a medicament, in particular to treat multiple sclerosis.

In another aspect, the invention discloses the use of a sIFNbeta-IgGFcmut according to the invention in the manufacture of a medicament for the prevention and/or treatment of hairy cell leukaemia, Kaposi's sarcoma, chronic myelogenous leukaemia, multiple sclerosis, hepatitis A, hepatitis B, hepatitis C, HIV and/or infections caused by viruses selected from Epstein Barr, herpes virus and/or papilloma virus.

In another aspect, the invention provides a method of making a sIFNbeta-IgGFcmut according to the invention, the method comprising culturing a host cell, preferably a CHO cell, according to the invention and isolating the sIFNbeta-IgGFcmut according to the present invention.

### Detailed description of the invention

The present invention relates to a single IFN-beta fused to a mutated IgG Fc domain (hereafter referred to as sIFNbeta-IgGFcmut) which can be delivered through pulmonary, oral or subcutaneous uptake. The sIFNbeta-IgGFcmut of the present invention display reduced Fc effector functions, good bioactivity, while still maintaining high serum levels and half-life in the circulatory system.

In a first aspect, the invention discloses sIFNbeta-IgGFcmut containing two subunits, the first subunit comprising a mutated IgG Fc arm not linked (Fcmut arm) to a IFN-beta protein and the second subunit comprising a mutated IgG Fc arm linked to a single IFN-beta protein (IFNbeta-Fcmut arm) wherein the mutated IgG Fc domain (IgGFcmut) is an immunoglobulin gamma (IgG)-1 Fc domain wherein at least the positions 234, 235, 237, 330 and 331, according to EU index position as defined Kabat et al. (Kabat E.A.et al. 1991), have been mutated. The mutations introduced result in a lower ADCC and in a lower CDC. In a preferred embodiment the mutations are L234A, L235E, G237A, A330S and P331S. In order to avoid confusion in the numeration of the mutations' positions, in the following Table 1 it is reported a comparison between the amino acid positions of some SEQ ID NOs of the invention and the EU index:

**Table 1**

| Mutations | SEQ ID NO: 1 | SEQ ID NOs 3, 5 | SEQ ID NOs 8, 10 | SEQ ID NO: 14 | SEQ ID NO: 15 | EU index (Kabat) |
|---|---|---|---|---|---|---|
| L→A | 117 | 14 | 180 | 14 | 188 | 234 |
| L→E | 118 | 15 | 181 | 15 | 189 | 235 |
| G→A | 120 | 17 | 183 | 17 | 191 | 237 |
| A→S | 213 | 110 | 276 | 110 | 284 | 330 |
| P→S | 214 | 111 | 277 | 111 | 285 | 331 |
| N→A | 180 | 77 | 243 | 77 | 251 | 297 |

Different allotypes of IgG1 are found in the population. The heavy constant region of human IgG1 recited in SEQ ID NO: 1 is the allotype referred to as the "G1M (non-1) marker" in Uniprot (accession number P01857). The mutations described in the present invention can be made in all the different allotypes found in the population. In the present invention, the Fc domain was derived from the "G1M (non-1) marker" allotype (see Example 1), however the Fc domain can also be derived from any other allotype.

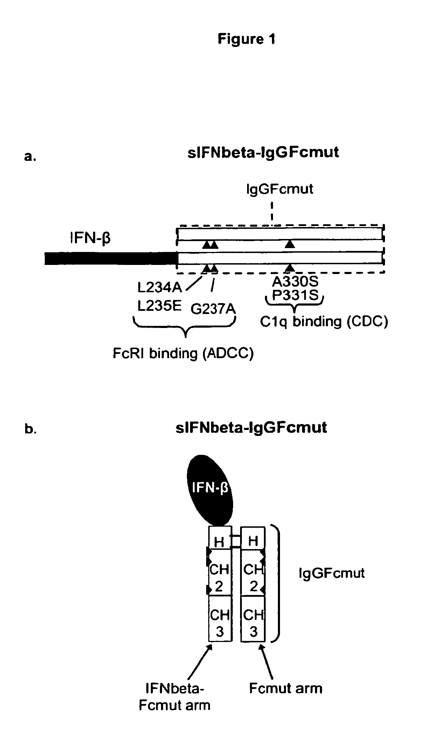

An immunoglobulin gamma (IgG)-1 Fc domain wherein at least the positions 234, 235, 237, 330 and 331, according to EU index position, have been mutated so as to result in a lower ADCC and in a lower CDC will be referred herein as IgGFcmut. The arm of the IgGFcmut not linked to a IFN-beta protein will be referred herein as Fcmut arm. The arm of the IgGFcmut linked to a single IFN-beta protein will be referred herein as IFNbeta-Fcmut arm (Figure 1). In a preferred embodiment the mutations in the IgGFcmut are L234A, L235E, G237A, A330S and P331S.

Herein, an Fc domain may be referred to as an Fc fragment, Fc-moiety or Fc region. Herein, the terms "Fc domain", "Fc fragment", "Fc region" or "Fc-moiety" are interchangeable and should be construed as having the same meaning.

The sIFNbeta-IgGFcmut of the invention displays similar bioactivity as the monomer-dimer protein of IFN-beta disclosed in WO 2005/001025. IFN-beta bioactivity is typically measured in an antiviral assay such as described in Example 5.

The siFNbeta-IgGFcmut of the invention reduces or inhibits antibody dependent cellular toxicity (ADCC) and complement dependent cytotoxicity (CDC). For example, the sIFNbeta-IgGFcmut of the invention reduces or inhibits binding to the Fc receptors FcγRI, CD64, IIA, IIB, IIIA and/or IIIB, and/or reduces or inhibits binding to C1q. Moreover the sIFNbeta-IgGFcmut Qf the invention keeps the binding properties (or affinity) for the FcRn. Reduced or inhibited ADCC and CDC, While keeping the FcRn binding of the sIFNbeta-IgGFcmut of the invention are conferred by the five mutations present in the Fc domain. The invention provides sIFNbeta-IgGFcmut which exhibit the same bioactivity and the same binding properties for the FcRn, with reduced or inhibited ADCC and CDC.

The sIFNbeta-IgGFcmut of the invention may be administered by inhalation or' by subcutaneous administration.

The high serum levels and half-life in the circulatory system (extended residence time in body fluids) of the sIFNbeta-IgGFcmut of the present invention make the subcutaneous route a preferred way of administration.

The sIFNbeta-IgGFcmut of the invention can be purified by using protein A or protein G affinity chromatography.

The sIFNbeta-IgGFcmut according to the invention contains 5 mutations in the Fc domain. In this embodiment, ADCC and CDC are significantly inhibited or at least significantly reduced.

The invention describes inter alia a sIFNbeta-IgGFcmut containing two subunits, the first subunit comprising a mutated Fc arm of an IgG Fc domain not linked (Fcmut arm) to a IFN-beta protein and the second subunit comprising a mutated Fc arm of the same IgG Fc domain linked to a single IFN-beta protein (IFNbeta-Fcmut arm) wherein the mutated IgG Fc domain (IgGFcmut) is an immunoglobulin gamma (IgG)-1 Fc domain wherein at least the positions 234, 235, 237, 330 and 331, according to EU index position, have been mutated. The mutations introduced result in a lower ADCC and in a lower CDC. In a preferred embodiment the mutations are L234A, L235E, G237A, A330S and P331S.

In a preferred embodiment the Fc domain does not contain the mutation N→A at the amino acid residue corresponding to EU index position number 297. In one embodiment, the invention discloses a sIFNbeta-IgGFCmut containing two subunits, the first subunit comprising a mutated IgG Fc arm not linked (Fcmut arm) to a IFN-beta protein and the second subunit comprising a mutated IgG Fc arm linked to a single IFN-beta protein (IFNbeta-Fcmut arm) wherein the Fc domain is the IgGFcmut and wherein the IFNbeta-Fcmut arm comprises a mutated IgG Fc arm that is directly linked to IFN-beta. In this embodiment, the sIFNbeta-IgGFcmut does not contain any linker between the IgGFcmut and IFN-beta.

In another embodiment, the invention describes a sIFNbeta-IgGFcmut containing two subunits, the first subunit comprising a mutated IgG Fc arm not linked (Fcmut arm) to a IFN-beta protein and the second subunit comprising a mutated IgG Fc arm linked to a single IFN-beta protein (IFNbeta-Fcmut arm) wherein the Fc domain is the IgGFcmut and wherein the IFNbeta-Fcmut arm comprises a mutated IgG Fc arm that is not directly linked to IFN-beta.

In a further preferred embodiment the IgGFcmut does not include the mutation N→A at the amino acid residue corresponding to EU index position number 297. Therefore the IgGFcmut according to the invention presents at position 297 the amino acid N. One of the sIFNbeta-IgGFcmut disclosed herein contain a linker between the IgGFcmut and IFN-beta.

The IgG1 Fc domain contains at least five mutations selected from L234A, L235E, G237A, A330S and P331S, according to EU index position or at least these five mutations at the equivalent positions of any other Fc domain. Thus, the above mutations can be made in the Fc domain of any IgG1 allotype.

The three mutations at 234 (e.g. L234A), 235 (e.g. L235E) and 237, (e.g. G237A) are designed so at to reduce or inhibit ADCC; the two mutations at 330 (e.g.A330S) and 331 (e.g.P331S) to reduce or inhibit CDC.

In one embodiment, the Fc domain comprises or consists of the CH₂ domain and/or the CH₃ domain and optionally the CH₁ domain, hinge region or fragment thereof, or any combination thereof. Preferably, the Fc domain comprises or consists of the hinge region or fragment thereof, the CH₂ domain and the CH₃ domain. In one embodiment, the Fc domain comprises or consists of the CH₂ domain and the CH₃ domain.

A so-called "hinge region" as defined in the present invention is a specific region located between the CH₁ and CH₂ domains. The hinge region is particularly sensitive to proteolytic cleavage; such proteolysis yields two or three fragments depending on the precise site of cleavage.

In one embodiment, the fragment of the hinge region consists of 1, 2, 3, 4, 5, 6, 7, 8, 9, 10 or 11 amino acids.

In another embodiment, the fragment of the hinge region consists of 7 amino acids. Preferably, the hinge region comprises or consists of SEQ ID NO: 2 (DKTHTCP).

More preferably, the IgGFcmut is composed by the Fcmut arm that comprises or consists of SEQ ID NO: 3 and by the amino acid portion from residue 167 to 393 of the IFNbeta-Fcmut arm that comprises or consists of SEQ ID NO: 8 (Figure 1). SEQ ID NO: 3 contains the mutations L14A, L15E, G17A, A110S and P111S that correspond, respectively, to IgG1 mutations L234A, L235E, G237A, A330S and P331S, according to EU index position. SEQ ID NO: 8 contains the mutations L180A, L181E, G183A, A276S and P277S that correspond, respectively, to IgG1 mutations L234A, L235E, G237A, A330S and P331S, according to EU index position. One of the Fc domain disclosed herein may also be further modified in order to reduce or inhibit effector functions.

The following Fc mutations, according to EU index positions, can be further introduced in the IgG1 Fc domain:
T250Q and/or M428L,
M252Y, S254T, T256E, H433K and/or N434F,
E233P, L234V, L235A, AG236, A327G, A330S and/or P331S,
E333A and/or K322A.

The T250Q and/or M428L mutations may increase serum half-life of the proteins.

The M252Y, S254T, T256E, H433K and/or N434F mutations may increase serum half-life of the proteins.

The E233P, L234V, L235A, AG236, A327G, A330S and/or P331S mutations may reduce or inhibit ADCC and CDC

Further Fc mutations e.g. may be the substitutions at EU index positions selected from 330, 331, 234, or 235, or combinations thereof. Furthermore, the cysteine residue at EU index position 220 may also be replaced with a serine residue, eliminating the cysteine residue that normally forms disulfide bonds with the immunoglobulin light chain constant region.

In one embodiment, a sIFNbeta-IgGFcmut disclosed herein further comprises a linker between the Fc domain and the cytokine. In one embodiment, the linker is a peptide which can be as short as 1 to 3 amino acid residues in length or longer, for example, 13 amino acid residues in length.

The linker may be a tripeptide of the sequence EFM (Glu-Phe-Met), for example, or a 13-amino acid linker sequence comprising EFGAGLVLGGQFM (Glu-Phe-Gly-Ala-Gly-Leu-Val-Leu-Gly-Gly-Gln-Phe-Met) introduced between the sequence of the cytokine and the immunoglobulin sequence. Preferably, the linker comprises or consists of SEQ ID NO: 13 (EFAGAAAV).

Preferably, the IFN is human IFN-beta (IFN-β), more preferably a recombinant human IFN-beta. Interferon beta-1a is a purified 166 amino acid glycoprotein with a molecular weight of approximately 22,500 Daltons. It is produced by recombinant DNA technology using genetically engineered Chinese Hamster Ovary cells into which the human interferon beta gene has been introduced.

Preferably, the IFN is a recombinant human IFN-beta which has a CHO cell-derived glycosylation. Preferably, both the IFN-β moiety and the Fc chains are gylcosylated. More preferably, the IFN-beta consists of SEQ ID NO: 7.

Rebif^{®} and Avonex^{®} are two examples of commercial preparations of CHO derived interferon beta-1a.

Betaseron^{®} is an example of commercial preparation of *E*. *coli* derived interferon beta-1b.

The first subunit of sIFNbeta-IgGFcmut according to the present invention comprising a mutated IgG Fc arm not linked (Fcmut arm) to a IFN-beta protein comprises or consists of SEQ ID NO: 3. SEQ ID NO: 3 with its signal peptide (signal peptide of mouse Igk light chain-see Example 1) is recited in SEQ ID NO: 5.

The second subunit of sIFNbeta-IgGFcmut according to the present invention comprising a mutated IgG Fc arm linked to a single IFN-beta protein (IFNbeta-Fcmut arm) comprises or consists of SEQ ID NO: 8. SEQ ID NO: 8 with its signal peptide (IFN-beta signal peptide) is recited is SEQ ID NO: 10.

The sIFNbeta-lgGFcmut according to the present invention is the protein herein referred to as "Compound 1". Compound 1 is the sIFNbeta-IgGFcmut of the invention and consists of SEQ ID NO: 3 and SEQ ID NO: 8. Compound 1 has five mutations in its Fc domain to inhibit ADCC and CDC activities (numbering according to standard human IgG heavy chain domain or EU index position): L234A (L14A of SEQ ID NOs 3 and 5; L180A of SEQ ID NOs 8 and 10), L235E (L15E of SEQ ID NOs 3 and 5; L181E of SEQ ID NOs 8 and 10), G237A (G17A of SEQ ID NOs 3 and 5; G183A of SEQ ID NOs 8 and 10), A330S (A110S of SEQ ID NOs 3 and 5; A276S of SEQ ID NOs 8 and 10) and P331S (P111S of SEQ ID NOs 3 and 5; P277S of SEQ ID NOs 8 and 10). The Fc domain of Compound 1 is derived from the "G1M (non-1) marker" allotype (see Example 1).

In another aspect, the invention provides a polynucleotide, e.g. a DNA, encoding a sIFNbeta-IgGFcmut according to the present invention, vectors and more particularly expression vectors containing said polynucleotide.

In one embodiment, the polynucleotide encodes SEQ ID NO: 3, SEQ ID NO: 8, SEQ ID NO: 5 or SEQ ID NO: 10. Preferably, a polynucleotide comprises or consists of SEQ ID NO: 4 encoding the first subunit of sIFNbeta-IgGFcmut (Fcmut arm). Preferably, a polynucleotide comprises or consists of SEQ ID NO: 9 encoding the second subunit of sIFNbeta-IgGFcmut (IFNbeta-Fcmut arm). Preferably, a polynucleotide comprises or consists of SEQ ID NO: 6 encoding the first subunit of sIFNbeta-IgGFcmut (Fcmut arm) and a signal peptide (i.e. the signal peptide of mouse Igk light chain). Preferably, a polynucleotide comprises or consists of SEQ ID NO: 11 encoding the second subunit of sIFNbeta-IgGFcmut (IFNbeta-Fcmut arm) and a signal peptide (i.e. the signal peptide of human IFN-beta).

In one embodiment, a DNA according to the invention comprises a bi-directional promoter. Preferably, the bi-directional promoter is a mouse bi-directional CMV promoter.

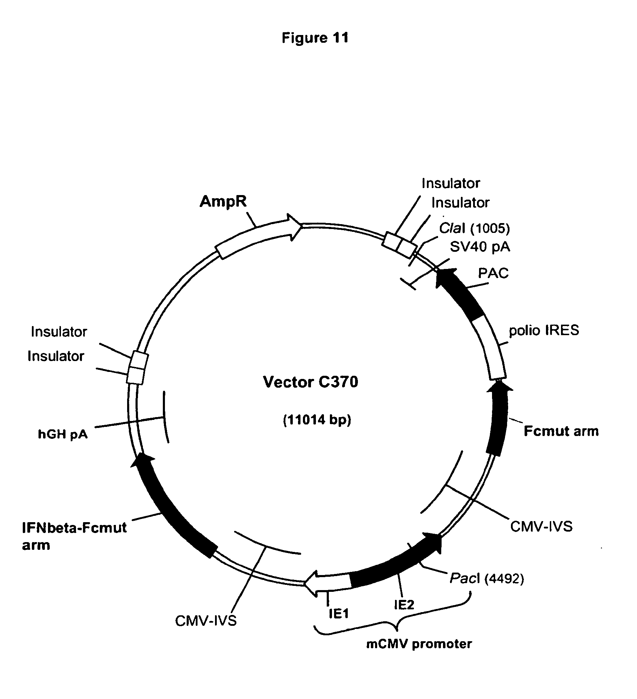

More preferably, a polynucleotide of the invention comprises or consists of SEQ ID NO: 12, herein also referred to as the C370 vector (Figure 11). Such vector contains the DNA sequences encoding for Compound 1 (i.e. SEQ ID NO: 6 and SEQ ID NO: 11).

In another aspect, the invention provides a host cell containing, e.g. as a result of a transfection, a vector according to the invention, a polynucleotide according to the present invention.

In another aspect, the invention provides a pharmaceutical composition comprising a sIFNbeta-IgGFcmut according to the present invention and its use as a medicament, in particular to treat multiple sclerosis.

Pharmaceutical compositions described herein are useful in the diagnosis, prevention, and/or treatment (local or systemic) of multiple sclerosis.

The term "treatment" within the context of this invention refers to any beneficial effect on progression of disease, including attenuation, reduction, decrease or diminishing of the pathological development after onset of disease.

The pharmaceutical compositions of the invention may be administered with a pharmaceutically acceptable carrier.

The term "pharmaceutically acceptable" is meant to encompass any carrier, which does not interfere with effectiveness of the biological activity of the active ingredient and that is not toxic to the host to which it is administered. For example, for parenteral administration, the active protein(s) may be formulated in a unit dosage form for injection in vehicles such as saline, dextrose solution, serum albumin and Ringer's solution.

In another aspect, the invention provides a pharmaceutical composition according to the invention for use as a medicament. In another aspect, the invention discloses a method of treating a disease in a patient, comprising administering to the patient a pharmaceutical composition according to the invention. Preferably, the disease is selected from hairy cell leukaemia, Kaposi's sarcoma, chronic myelogenous leukaemia, multiple sclerosis, hepatitis A, hepatitis B, hepatitis C, HIV and/or infections caused by viruses selected from Epstein Barr, herpes virus and/or papilloma virus. More preferably, the disease is multiple sclerosis. In one embodiment, the disease is Relapsing-Remitting Multiple Sclerosis (RRMS)

In another aspect, the invention discloses for the use of a siFNbeta-IgGFcmut according to the invention in the manufacture of a medicament for the prevention and/or treatment of hairy cell leukaemia, Kaposi's sarcoma, chronic myelogenous leukaemia, multiple sclerosis, hepatitis A, hepatitis B, hepatitis C, HIV and/or infections caused by viruses selected from Epstein Barr, herpes virus and/or papilloma virus.

In a first use according to the invention, a pharmaceutical composition according to the invention is administered pulmonary.
In a second use according to the invention, a pharmaceutical composition according to the invention is administered intranasally.
In a third use according to the invention, a pharmaceutical composition according to the invention is administered by inhalation.
In a fourth use, according to the invention, a pharmaceutical composition according to the invention is administered orally.
In a fifth use according to the invention, a pharmaceutical composition according to the invention is administered intravenously or intramuscularly..
In a preferred embodiment, in a use according to the invention, a pharmaceutical composition according to the invention is administered subcutaneously.

A pharmaceutical composition according to the invention is administered according to any one of the routes described above daily or every other day.

If administered subcutaneously or intramuscularly, a pharmaceutical composition according to the invention is administered once, twice or three times per week, but more preferably every two weeks.

For parenteral (e.g. intravenous, subcutaneous, intramuscular) administration, a pharmaceutical composition of the invention can be formulated as a solution, suspension, emulsion or lyophilised powder in association with a pharmaceutically acceptable parenteral vehicle (e.g. water, saline, dextrose solution) and additives that maintain isotonicity (e.g. mannitol) or chemical stability (e.g. preservatives and buffers). The formulation is sterilized by commonly used techniques.

The active ingredients of the pharmaceutical composition according to the invention can be administered to an individual in a variety of ways. The routes of administration may include intradermal, transdermal (e.g. in slow release formulations), intramuscular, intraperitoneal, intravenous, subcutaneous, epidural, topical, oral routes and preferably by aerosol administration, intranasal route or inhaled. Any other therapeutically efficacious route of administration can be used, for example absorption through epithelial or endothelial tissues or by gene therapy wherein a DNA molecule encoding the active agent is administered to the patient (e.g. via a vector), which causes the active agent to be expressed and secreted *in vivo.* In addition, a pharmaceutical composition according to the invention can be administered together witch other components of biologically active agents such as pharmaceutically acceptable surfactants, excipients, carriers, diluents and vehicles.

Preferably, in a use according to the invention, a pharmaceutical composition according to the invention is delivered by a spray device, aerosol spray or inhaler. In one embodiment, a pharmaceutical composition of the invention can be administered using recognized devices. Examples comprising single vial systems include auto-injector or pen-injector devices for delivery of a solution such as Rebiject^{®}.

In another aspect, the invention describes a device, wherein the device comprises a pharmaceutical composition according to the invention. Preferably, the device is a spray device.

The dosage administered, as single or multiple doses, to an individual will vary depending upon a variety of factors, including pharmacokinetic properties, the route of administration, patient conditions and characteristics (sex, age, body weight, health, size), extent of symptoms, concurrent treatments, frequency of treatment and the effect desired.

The expression "multi-dose use" is intended to include the use of a single vial, ampoule or cartridge of a pharmaceutical composition, a sIFNbeta-IgGFcmut of the invention for more than one injection and/or inhalation, for example 2, 3, 4, 5, 6 or more injections and/or inhalations. The injections and/or inhalations are preferably made over a period of at least at or about 12 hours, 24 hours, 48 hours, etc., preferably up to a period of at or about 12 days. The injections and/or inhalations may be spaced in time, for example, by a period of 6, 12, 24, 48 or 72 hours.

In one embodiment, in a use according to the invention, a pharmaceutical composition according to the invention is administered at a dosage of about 30 to 120 µg of siFNbeta-IgGFcmut per person per day.

Preferably, in a use according to the invention, a pharmaceutical composition according to the invention is dosed at 1, 5, 8, 10, 12, 15, 20 or more µg of sIFNbeta-IgGFcmut /kg per inhalation.

Preferably, in a use according to the invention, a pharmaceutical composition according to the invention is administered at a dosage of about 30 to 10000 µg of sIFNbeta-IgGFcmut per person per day, or about 60 to 2000 µg of sIFNbeta-IgGFcmut per person per day or about 90 to 1000 µg of sIFNbeta-IgGFcmut per person per day.

In one embodiment, in a use according to the invention, a pharmaceutical composition according to the invention is dosed at least at 107, more preferably at least at 157 µg of sIFNbeta-IgGFcmut s.c. per administration.

Instead of referring to the weight (µg) of sIFNbeta-IgGFcmut, the dosage may be calculated in equivalent amounts of IFN-beta.

In another aspect, the invention provides a method of making a sIFNbeta-IgGFcmut according to the invention, the method comprising culturing a host cell according to the invention and isolating the sIFNbeta-IgGFcmut according to the present invention.

The foregoing description of the specific embodiments will so fully reveal the general nature of the invention that others can, by applying knowledge within the skill of the art (including the contents of the references cited herein), readily modify and/or adapt for various application such specific embodiments, without undue experimentation, without departing from the general concept of the present invention. Therefore, such adaptations and modifications are intended to be within the meaning of a range of equivalents of the disclosed embodiments, based on the teaching and guidance presented herein. It is to be understood that the phraseology or terminology herein is for the purpose of description and not of limitation.

### Description of the figures

Fig. 1 describes an embodiment of the invention and is a schematic representation of the two subunits of a sIFNbeta-IgGFcmut. Fig. 1a shows the five mutations in the Fc arms of a sIFNbeta-IgGFcmut numbered according to EU index position and their effects. Fig. 1b represents a sIFNbeta-IgGFcmut and clarifies the names of the subunits.

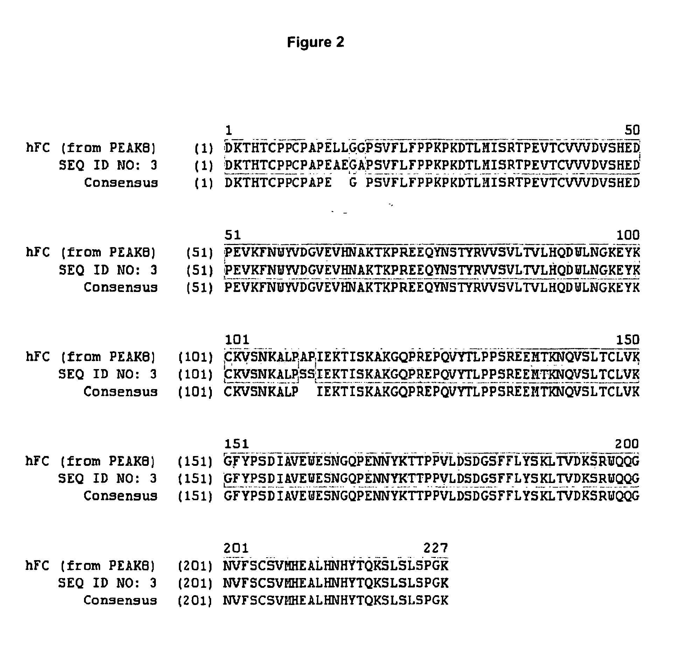

Fig. 2 describes an embodiment of the invention and represents a sequence alignment of the amino acid mutations introduced into the Fcmut arm of Compound 1 (i.e. SEQ ID NO: 3) by site-directed mutagenesis in comparison with the equivalent portion of the Fc domain derived from a cDNA clone obtained from an MGC/Image consortium clone (hFC from PEAK8; a "G1M (non-1) marker" allotype). The numeration is based on SEQ ID NO: 3. The first three mutations reduce ADCC while the last two reduce CDC. The mutations L14A (i.e. L234A according to EU index), L15E (i.e. L235E according to EU index) and G17A (i.e. G237A according to EU index) eliminate binding to Fc receptors FcγRI, IIA, IIB and IIIA, while mutations A110S (i.e. A330S according to EU index) and P111S (i.e. P331S according to EU index) eliminate binding to the complement cascade initiator protein C1q.

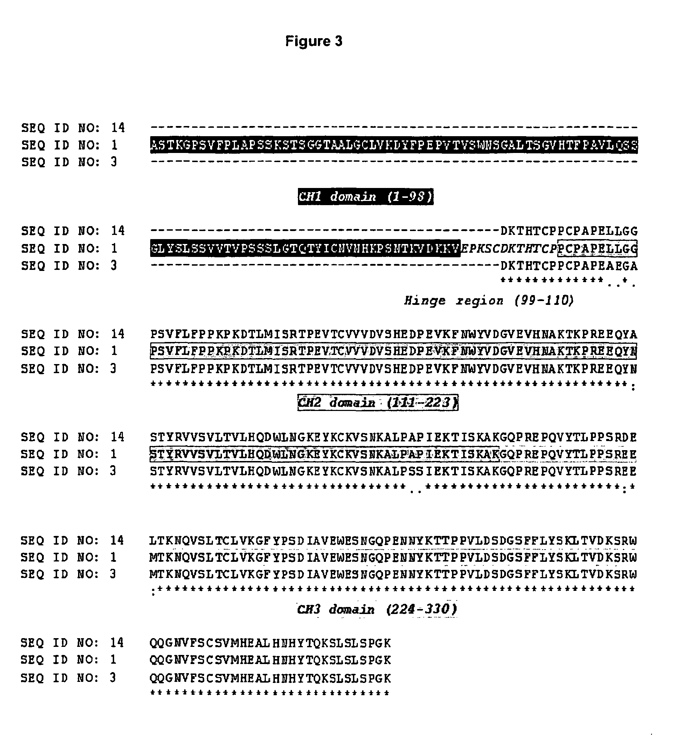

Fig. 3 describes an embodiment of the invention and represents a sequence alignment between the Fcmut arm of Compound 1 (i.e. SEQ ID NO: 3), the corresponding portion of Compound 2 (i.e. SEQ ID NO: 14. Compound 2 is a monomer-dimer protein that contains a linker sequence of 8 amino acids between IFN-β and the Fc domain and contains only 1 mutation in the Fc domain) and the heavy chain constant region of the "G1M (non-1) marker" allotype (i.e. SEQ ID NO: 1). The numeration is based on SEQ ID NO: 1. "." indicates the amino acids positions wherein SEQ ID NO: 3 differs from SEQ ID NOs 1 and 14. ":" indicates the amino acid positions wherein SEQ ID NO: 14 differs from SEQ ID NOs 1 and 3.

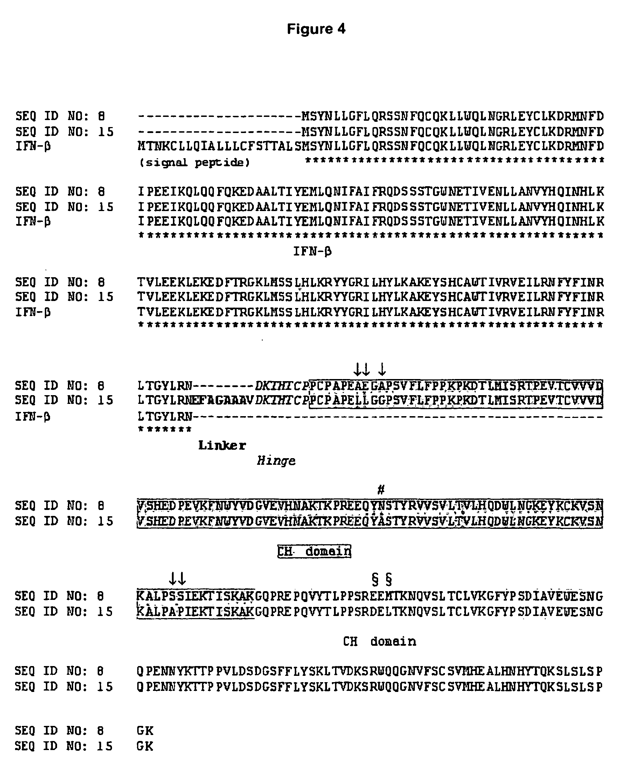

Fig. 4 describes an embodiment of the invention and represents a sequence alignment between the IFNbeta-Fcmut arm of Compound 1 (i.e. SEQ ID NO: 8), the corresponding portion of Compound 2 (i.e. SEQ ID NO: 15) and the human IFN-β (UniProt accession number P01574). "↓" indicate the positions wherein IFNbeta-Fcmut arm has been mutated. "#" indicates the position wherein SEQ ID NO: 15 has been mutated. "§" indicate the positions that characterize the two different allotypes on which Compound 1 and 2 have been constructed.

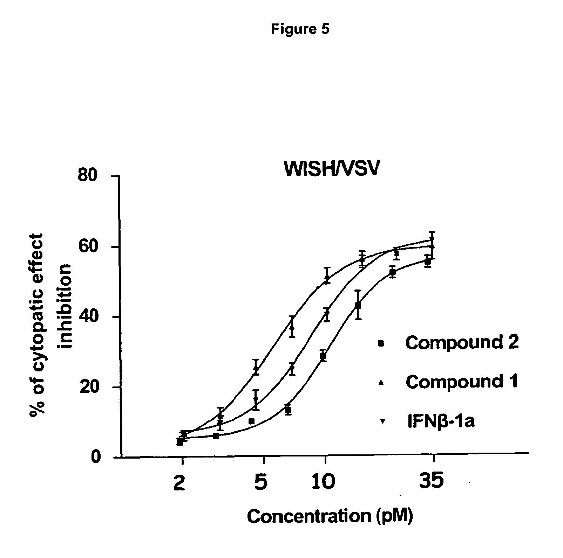

Fig. 5 describes an embodiment of the invention and is an antiviral assay WISH/VSV method [WISH cells from human amnion cell line against the cytopathic effect (CPE) of the vesicular stomatitis virus (VSV)]. The curves correspond to the dose-response curves of Compound 1, Compound 2 and recombinant human IFN-β-1a. Data are reported as percentage of cytopatic effect inhibition.

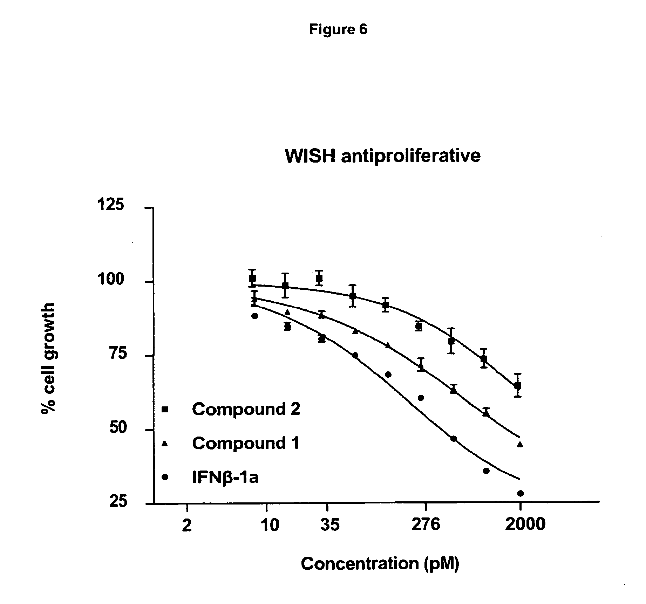

Fig. 6 describes an embodiment of the invention and is an antiproliferative assay of WISH cells. The curves correspond to the dose-response curves of Compound 1, Compound 2 and recombinant human IFN-β-1a. Data are reported as percentage of cell growth.

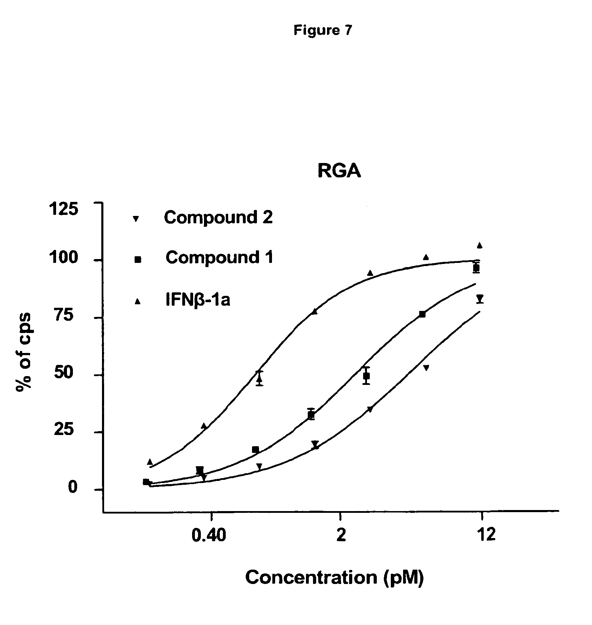

Fig. 7 describes an embodiment of the invention and is a Reporter Gene Assay (RGA) on Vero cells. The curves correspond to the dose-response curves of Compound 1, Compound 2 and recombinant human IFN-β-1a. Data are reported as percentage of count per second (cps).

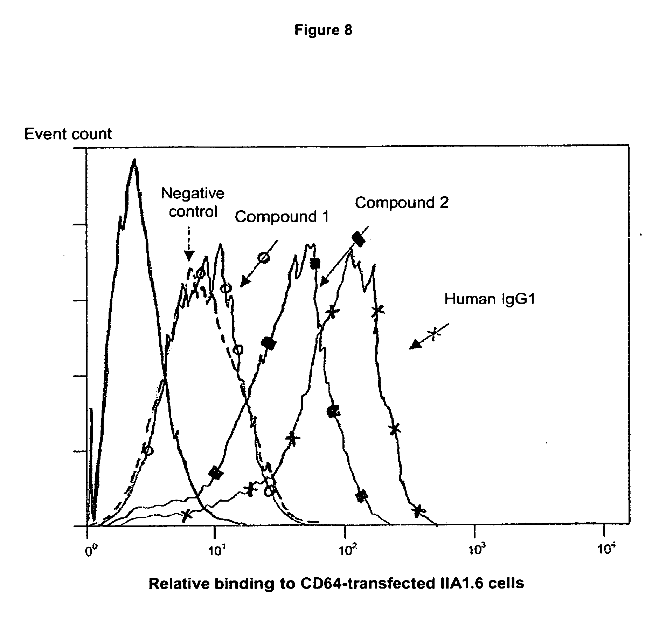

Fig. 8 describes an embodiment of the invention and shows the relative binding of Compound 1 and Compound 2 to CD64-transfected IIA1.6 cells.

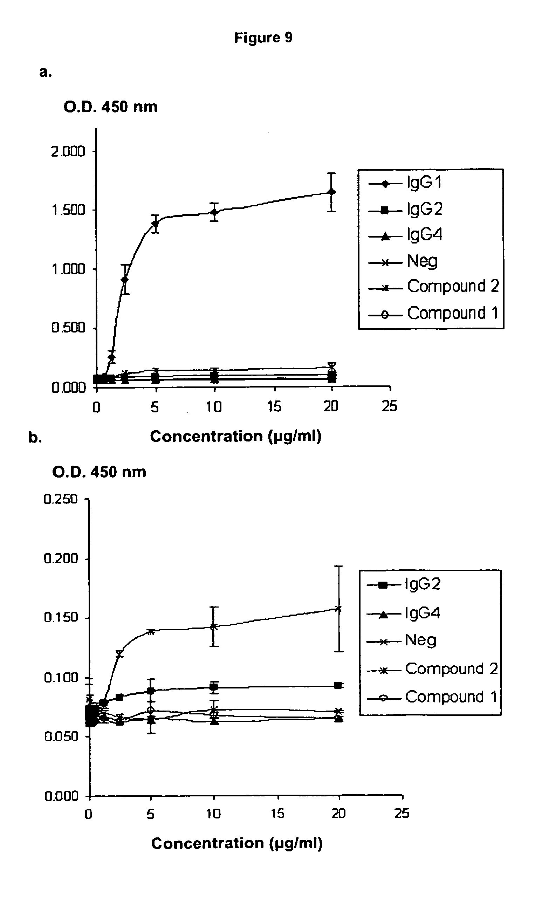

Fig. 9 describes an embodiment of the invention and shows the binding of C1q to Compound 1 and Compound 2. Fig. 9a includes IgG1. Fig. 9b excludes IgG1.

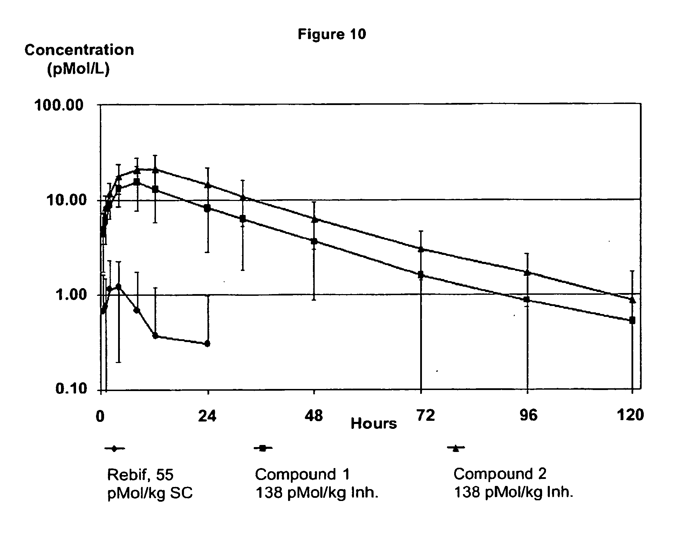

Fig. 10 describes an embodiment of the invention and displays the Mean (SD) IFN-β, Compound 1 or Compound 2 serum profiles. The curves correspond to: IFN-β serum levels after Rebif^{®} subcutaneous (SC) administration 55 pMol/kg; Compound 1 serum levels after administration of Compound 1 by inhalation route (Inh.) at the nominal deposited dose of 138 pMol/kg; and Compound 2 serum levels after administration of Compound 2 by inhalation route at the nominal deposited dose of 138 pMol/kg.
Fig. 11 describes an embodiment of the invention and displays the C370 expression vector used to produce Compound 1.

In the following the present invention shall be illustrated by means of the following examples

### Examples

### Analytical protocols used for characterization

### 1. RP-HPLC

| | |
|---|---|
| HPLC: | Agilent 1100 Series system (Agilent Technologies) |
| Column: | Aquapore BU-300 30x2.1mm (Brownlee 0711-0062) |
| Solvent A: | 0.1% TFA in water |
| Solvent B: | 0.1% TFA in 90 % Acetonitrile |
| Gradient: | ProtC4 |

### Sample preparation:

15 µg injected
Dissolve protein in solvent A to a final volume of 100 µl

### 2. SDS-PAGE

| | |
|---|---|
| Gel: | NuPage 4-12% Bis-Tris (Invitrogen NP0322) |
| Buffer: | NuPage MES SDS running buffer (Invitrogen NP0002) 10x concentrated sample buffer. |
| | add 10 mM DTT for reduced conditions |
| Run conditions: | 200 V 40 min |

### Sample preparation:

10 µg loaded
Dissolve protein in 10x sample buffer to a final volume of 20 µl
Heat for 5 min at 95°C
Stain in 0.1% Coomassie Blue, 30% Methanol, 10% Acetic Acid, 45 min.
Distain in 30% Methanol, 10% Acetic Acid

### 3. IEF

| | |
|---|---|
| Gel: | pH 3-10 IEF gel (Invitrogen ECC6655A) |
| Buffers: | IEF Anode buffer (Invitrogen LC5300 |
| | IEF Cathode buffer (Invitrogen LC5310) |
| | IEF pH 3-10 sample buffer (Invitrogen LC5311) |

### Sample preparation:

10 µg loaded
Dilute sample with one volume of IEF sample buffer (Do not heat samples)

| | |
|---|---|
| Run conditions: | 1h at 100V |
| | 1h at 200V |
| | 2h at 400V |

Fix the gel in 30% Methanol, 10% Acetic acid 2 × 30 min.
Stain in 0.1% Coomassie Blue, 30% Methanol, 10% Acetic Acid, 15 min.
Distain in 30% Methanol, 10% Acetic Acid

### 4. Western Blot

| | |
|---|---|
| Blocking solution: | PBS/0.1% Triton X100, 5% Milk |
| Washing solution: | PBS/0.1% Triton X100 |
| Antibody solution: | Dilute antibody in washing solution. |

Block 1-2h at RT or O/N at 4°C
Wash 3 x 5min
Incubate with the 1^{st} antibody 1h at RT
Wash 30 min
Incubate with the 2^{nd} antibody 1 h at RT
Wash at least 1h.
Western blotting detection by ECL: follow procedure provided with reagent.

### Antibodies

anti-Fc = Goat anti-human IgG, Fc fragment HRP (10000xdil.) (Jackson 109-036-098)
anti-IFN-beta =
   1^{st} antibody: anti-IFN-beta mab 117.1 0.1 µg/ml
   2^{nd} antibody: goat anti-mouse IgG *HRP* (1000x dil.) (Dako P0447)

### 5. Deglycosylation

N-Glycosidase F (Roche 11 365 193 001)
10 µg sIFNbeta-IgGFcmut in Phosphate buffer pH 7.5 / 10 % Acetonitrile
add 5U PNGase F
Incubate O/N at 37°C

### 6. Reduction after deglycosylation

Incubation of sample in 4M guanidine HCL, 10 mM DTT, 1h at 56°C

### 7. N-Terminal sequencing

200 pmoles loaded
Sample is loaded on a Prosob cartridge (ABI 402052) before to be sequenced.
(The Prosorb cartridge is designed for the rapid concentration and clean up of dilute protein samples onto a matrix suitable for sequencing (PVDF membrane)).
Equipment:
Model 494 Procise micro sequencing system on line with a Microgradient 140C HPLC (Applied Biosystems).

### 8. Amino acid analysis

Amino acid analysis is carried out by gas-phase hydrolysis under vacuum in nitrogen.
Atmosphere for 24 h in 6N HCl containing 1 mg/ml phenol at 112°C.
Alpha Amino butyric acid (AAbA) is included in the hydrolysates as an internal standard.
Samples are analyzed in duplicate.
Hydrolysed amino acids are quantified using the Waters AccQ.Tag chemistry package.

### Waters AccQ_{.}Tag

This is a pre-column derivatization technique.
AA derivatives are separated by RP-HPLC and quantified by fluorescent detection.
A 3 level calibration is made (20, 50 and 100 picomoles)

### Material

### i) Protein Hydrolysis:

Reaction vials assembly (Waters WAT007363)
Sample tubes (Waters WAT007571)
Pico.Tag workstation (Waters WAT007370)

### ii) AAA Derivatization, Separation and Quantitation:

HPLC: Agilent 1090 Series system (Agilent Technologies)
Detector: FLD G1321A Agilent 1100 series (Agilent Technologies)
Excitation: 250 nm Emission: 395 nm
Column: AccQ.Tag C18 4 µm 3.9 x 150mm (Waters WAT052825)
Solvent A: 900 ml H₂O + 90 ml AccQ.Tag Eluent A Concentrate (WAT052890)
Solvent B: 60% Acetonitrile (Baker 9017) in H2O

### HPLC Gradient:

| Time [min] | %B | Flow | [ml/min] |
|---|---|---|---|
| | 0.50 | 2.0 | 1.000 |
| | 15.00 | 7.0 | 1.000 |
| | 19.00 | 10.0 | 1.000 |
| | 32.00 | 33.0 | 1.000 |
| | 33.00 | 33.0 | 1.000 |
| | 34.00 | 100.0 | 1.000 |
| | 37.00 | 100.0 | 1.000 |
| | 38.00 | 0.0 | 1.000 |
| | 50.00 | 0.0 | 1.000 |

### 9. MALDI-TOF-MS

Mass spectrometric analyses were performed on a Voyager DE-PRO MALDI-TOF mass spectrometer (Applied Biosystems) operated in the linear mode.
Sinapinic acid was used as energy absorbing molecule.
Purified protein samples were suspended in a 0.1% TFA solution at 1 mg/ml concentration. One µg protein was spotted on the sample target. One µl of a saturated sinapinic acid solution (50% acetonitrile in H₂O plus 0.1% TFA) was added on top of the protein droplet and allowed to dry at room temperature.
Mass calibration was performed for each analysis using calibration standards from ABI.

### 10. SE-HPLC Method

| | |
|---|---|
| HPLC: | Waters Alliance 2695 |
| Mobile phase: | 50mM Sodium Phosphate + NaCl 500 mM at final pH 7.5 |
| Column: | Superdex 200 10/300GL at RT. Column must be used in vertical position. |
| Flow rate: | from 500 µL/min to 750 µL/min (with max pressure below 210psi) |
| Autosampler: | Waters Alliance 2695 at 5°C |
| Detection: | Waters Fluorescence 2475 (with emulation 474 on Empower Software), with excitation 280 nm, emission 348 nm, window 18 nm, Gain 100, Attenuation 1 |

### Example 1

The protein of the invention is the sIFNbeta-IgGFcmut referred to as Compound 1. In Compound 1 one subunit (IFNbeta-Fcmut arm, SEQ ID NO: 8) is the fusion product of IFN-β and a mutated Fc arm of the human IgG1 Fc domain. The other subunit (Fcmut arm, SEQ ID NO: 3) is identical to the Fc portion alone of the first subunit. The sequence of the Fc portion present in both subunits has been modified by substituting in a "G1M (non-1) marker" allotype five amino acids in order to reduce immunoglobulin effector functions (Figures 1, 3 and 4). A monomer-dimer protein of IFN-beta disclosed in WO 2005/001025, herein referred to as "Compound 2", has been prepared for comparison purposes. Compound 2 contains a linker sequence of 8 amino acids between IFN-β and the Fc domain and contains only 1 mutation (i.e. N297A according to the EU index position) in the Fc domain compared with the Fc sequence derived from a different IgG1 allotype (Figure 4).
The subunit of Compound 2 not linked to IFN-beta is recited in SEQ ID NO: 15. The subunit of Compound 2 linked to IFN-beta through a linker of 8 amino acids is recited in SEQ ID NO: 16.
The manufacturing of Compound 1 has been established by the generation of a clone in CHO-S cells cultivated in the absence of serum. CHO-S cells are derived from Chinese hamster ovaries and adapted to serum-free suspension culture (Invitrogen/Gibco, La Jolla, CA).

### 1. Cloning

The expression vector C370 (Figure 11; the sequence of the C370 vector is recited in SEQ ID NO: 12) used to produce Compound 1 contains a bi-directional murine CMV promoter (IE1 and IE2). The IFN-beta coding sequence was derived by PCR from human genomic DNA. An IgG1 heavy chain cDNA clone (a "G1M (non-1) marker" allotype) was obtained from an MGC/Image consortium clone derived from a human spleen cDNA library. The mutated human IgG1 Fc domain (IgGFcmut) was derived by PCR from a PEAK8 plasmid containing the Fc domain from the "G1 M (non-1) marker" allotype, wherein the Fc domain was mutated in two steps using the Quik change II mutagenesis system (Stratagene). Several single amino acid substitutions were introduced into the Fc domain to mutate amino acids critical for mediating ADCC/CDC (Figures 2 to 4). In the first step, the sequence encoding amino acids ¹¹⁷LLGG¹²⁰ of SEQ ID NO: 1 (i.e. ²³⁴LLGG²³⁷ of EU index) was mutated to ¹¹⁷AEGA¹²⁰. In the 2^{nd} step, the sequence encoding amino acids A213 (i.e. A330 of EU index) and P214 (i.e. P331 of EU index) of SEQ ID NO: 1 was mutated to S213 and S214. A cDNA encoding IFN-beta including its cognate signal peptide sequence, in frame with a mutated Fc arm of the sIFNbeta-IgGFcmut was generated by overlapping PCR and subcloned downstream of the IE1 promoter. A cDNA encoding the signal peptide sequence of mouse Igk light chain fused to the coding sequence of the other mutated Fc arm of the siFNbeta-IgGFcmut was generated by PCR and subcloned downstream of the IE2 promoter (Figure 11).

### 2. Expression vector construction

The IFNbeta-Fcmut arm fusion gene was thus generated by recombinant PCR and cloned into an expression vector for high-level constitutive expression into CHO cells. The final vector C370 contains a mouse bi-directional CMV promoter.

The selection pressure was linked to the coding sequence of the Fcmut arm in order to have a favorable balance of IFN-beta dimer to sIFNbeta-IgGFcmut ratio, thus enabling an easier elimination of IFN-beta dimer during DSP purification. The mouse CMV bi-directional promoter drives the expression of the two chains of Compound 1. The selection is linked to the Fcmut arm expression using a poliovirus internal ribosome entry site (IRES). The expression unit is shielded from repression of nearby chromatin at the site of random integration by using insulator sequences.

### 3. Preparation of DNA for transfection

Plasmid DNA for transfection was prepared to ensure Stable Transfection Quality. DH5α bacteria (Invitrogen, Cat. #18265-017) were transformed with the original plasmid DNA according to the supplier's protocol, and selected in the presence of 100 µg/ml of ampiciline. A single colony was picked; cells were grown over day in 1 ml of LB containing 100 µg/ml of ampiciline and amplified in a 100 ml culture over night. Plasmid DNA was isolated as described in the manual for the Nucleobond PC 500 EF kit (Macherey-Nagel, Cat. #740550). The quality of the isolated DNA was assessed by measuring the OD₂₆₀ₙₘ and OD₂₈₀ₙₘ and calculating the ratio OD₂₆₀ₙₘ/OD₂₈₀ₙₘ that has to be greater than 1.8. Plasmid identity was verified by digesting the DNA with 4 restriction enzymes in independent reactions. The coding regions for the Fcmut arm and for the IFNbeta-Fcmut arm were sequenced in both orientations prior to transfection. Furthermore, plasmid DNA was linearized with an appropriate enzyme cutting in the vector backbone.
The vector C370 was linearized with Clal (1.255 µg/µl).

### 4. Origin of host cell and standard cultivation procedures

The CHO-S cells were obtained from Invitrogen. Non-transfected CHO-S cells were grown in ProCHO5 medium (Bio Whittaker, Cat. #12-766Q) supplemented with 4.5 mM L-glutamine (Sigma, Cat. #G7513). Transfection was carried out in the above-described medium. For selection, ProCHO5 supplemented with 4.5 mM L-glutamine and 10 µg/ml puromycin (Sigma, Cat. #P-7255) was used. The cell lines were grown in suspension and were incubated at 37°C in a 5% CO₂ containing incubator in T75 flasks or in Erlenmeyer (135 ml) flasks shaken at 130 rpm. Generally cells were passaged by dilution to 0.2 x 10⁶ viable cells/ml 2-3 times a week.

### 5. Freezing, thawing and cultivation of cells

Usually 5-10 x 10⁶ viable cells were frozen per vial. The required amount of cells was centrifuged and resuspended in 0.5 ml of ProCHO5 medium. Next, 0.5 ml of ProCHO5 Freezing Medium (Bio Whittaker, Cat. #12-768E), supplemented with 15% DMSO (Sigma) was added. Cells were frozen at -80°C, and then transferred into a storage tank refrigerated with nitrogen.
For thawing, an ampoule was placed in a 37°C water bath. Next, cells were transferred to 10 ml of the pre-warmed ProCHO5 medium, centrifuged at 800g for 3 min, and then resuspended in fresh medium at 0.2 to 0.4 x 10⁶ cells/ml in a T75 flask.
Cells were passaged 2 or 3 times per week by diluting the culture to 0.2 x 10⁶ viable cells/ml. In T75 flasks the cells were cultured in 10-20 ml of medium and in Erlenmeyer flasks in 35 ml of medium.

### 6. Stable transfections

The day before transfection, CHO-S cells, growing in ProCHO5 medium supplemented with 4.5 mM L-glutamine, were diluted to 0.7 x 10⁶ viable cells/ml. Transfection was performed by electroporation (400V, 250µF), 12 mln CHO-S, 10 µg (8 µl) C370.

### 7. Selection

48 hours post-transfection cells were counted, spent medium was removed by centrifugation, and cells were then diluted to 0.5 x 10⁶ viable cells/ml in selection medium (ProCHO5 supplemented with 4.5 mM L-glutamine and 10 µg/ml of puromycin). The medium was changed every other day. Cell densities were monitored over time and, when the number of viable cells dropped below 0.1 x 10⁶ cells/ml, the cells were concentrated in a smaller volume. Otherwise, when the number of viable cells increased, cells were diluted to 0.4-0.5 x 10⁶ cells/ml. This procedure was repeated until the viability of the pool reached 90%. The pools obtained were then tested for productivity (titer and picogram per cell per day, pcd) and biological activity (bioassay), and were directly used to isolate protoclones.

### 8. Isolation of protoclones

Pools of stably transfected cells were diluted to 1 cell per well (in 70 µl) in selection medium supplemented with 0.3% (v/v) of 0.5% phenol red solution (Sigma, Cat. #P0290) and plated in 384 well plates with the help of a Multidrop dispensing robot (Thermo Labsystems). Two weeks after seeding, independent protoclones were picked up and re-arrayed in 96 well plates in 200 µl of appropriate medium devoid of selective agents. 300 protoclones were isolated for each pool. Cells were diluted once a week 20 fold, in 96 well format, with the help of a Beckman Biomek 2000 robot.

### 9. Isolation and evaluation of clones

The protoclone culture was diluted to a concentration of 0.4 cell per well (in 70 µl) in appropriate medium supplemented with 0.3% (v/v) of phenol red and then plated in 384 well plates with the help of a Multidrop dispensing robot. 4 to 5 days post seeding, the growth in each well was examined under the microscope, and the wells with only one growing clone were marked. To assure clonality, the number of wells with growing cells was counted and had to be below 33% of the whole plate. After 15 days of growth, selected clones were transferred in 24-well plates, expanded, and then 6 clones for each initial protoclone were further analysed for productivity with a quantitative Biacore assay. Titer and specific productivity were evaluated in 24-hour pulses in T75 flasks. The 4 best clones were chosen and further analyzed in shake flask experiments.
Alternatively to the Biacore method, RP-HPLC and SE-HPLC can be used as analytical methods.

### 10. Productivity measurement

Protein concentration and specific productivity were measured in 24-hour pulses. For this, cells were resuspended at 0.8 x 10⁶ viable cells/ml in 10 ml of fresh medium (T75 flask shaken at 60 rpm). After 24 hours, cells were counted and 1 ml of the culture was collected for testing the protein concentration by an IFN-β specific Biacore assay. Specific productivity (expressed as picogram per cell per day, pcd) was calculated as titer (mg/1) / average cell density between start and end of the pulse (reported as cells/ml) / pulse duration (1 day). In shake flask experiments, the pcd was calculated as a function of IVC over a defined period of time.

### 11. SDS-western blot analysis

Western blot analysis was performed on culture supernatants. In brief, protein samples were separated on gradient 8-18% acrylamide gels of 1 mm thickness, and electrophoresis was performed at constant current (30mA) at 4°C followed by transfer to a PVDF membrane. Anti-IFNβ or Fc monoclonal antibody was used for epitope detection.

### 12. Northern blot analysis

Total RNA was isolated from 8 x 10⁶ cells according to the RNeasy MiniKit protocol (Qiagen, Cat. #74104). Northern blots were prepared using the NorthernMax™-Gly kit (Ambion, Cat. #1946) according to the supplier's protocol. 100 ng of RNA were loaded per well, separated by agarose gel electrophoresis (1% agarose) and downwards transferred to a BrightStar-Plus membrane (Ambion, Cat. #10102) using the Turboblotter kit (Schleicher & Schuell, Cat. #10416328). 10 µg of RNA ladder (Invitrogen, Cat. #15620-016) were used as a size marker. RNA was immobilised by UV crosslinking (Stratalinker) and baking (10 min, 70°C). Probe labelling with DIG and hybridisation (at 68°C) were performed using the DIG Northern Starter Kit (Roche, Cat. #2039672) according to the supplier's protocol. 100 ng/ml of the RNA probe were used for hybridisation. Probe was prepared using standard *in vitro* transcription protocol with T3 RNA polymerase.
The pattern obtained was revealed by exposure of the membrane to a Hyper Film (Amersham Pharmacia Biotech, Cat. #RPN2103K) or by acquiring an image with Chemi Doc (BioRad).

### Example 2

### 1. Expression

To produce 60 mg of Compound 1, a generic culture process was applied at the 5L scale in bioreactors. The cell line (a clone) is CHO-S and was cultivated in the absence of serum, in "bolus fed-batch" mode (batch culture with single additions). Two runs were necessary to produce the required quantity.
The cells were amplified in shake flasks to create the inoculum of each bioreactor. Amplification went through 125 ml, 500 ml and 1000 ml shake flasks respectively filled with 30 ml, 200 ml and 500 ml of medium. The 5L bioreactor contained a final volume of 3L, including the volume of inoculum and the feeds.
The maintenance medium used at all stages was the ProCHO5 from Cambrex (Belgium) supplemented with 4.5 mM L-glutamine.
Two bolus feeds were applied. The first one, F1, contained 0.68 g/L Hypoxanthine 0.68 g/L, 0.194 g/L thymidine 0.56 g, 42 mg/L L-cysteine·HCl, 0.5 mM folic acid and 4.5 mM L-glutamine (final concentrations). F1 was applied at day 2 post-inoculation. Feed F2 was made of 4.5 mM L-glutamine with or without 5 g/L glucose (final concentrations). Glucose was added in F2 only when the amount of residual glucose in the medium was below 3 g/L.
The settings of the culture were as follows:
- temperature = 37°C, shifted down to 32°C at maximal cell density,
- agitation = 100 rpm, raised to 120-130 rpm after first feed,
- maximum pH = 7.3. Alcalinization was controlled by CO₂ addition.
- PO₂ = 50% of saturation.
Cell concentrations at inoculation and at harvest after 6 days of culture were respectively 4.0 x 10⁵ and 3.4 x 10⁶, with a final viability of about 72%.
The culture parameters were measured on-line and regulated. Off-line measurements included cell counts, assessment of viability by Trypan Blue, residual glucose concentration (YSI analyzer, Yellow Springs Instruments) and qualitative protein expression pattern over time.

### 2. Purification

A purification protocol has been developed that effectively isolates the sIFNbeta-IgGFcmut protein from other forms produced.
A purification process was established with the material generated by culturing an initial CHO pool. It was then directly scaled up and used to purify the cell culture supernatants from the protoclone.
Herein, the Fc-tagged molecules will be referred to as follows:
- "Free Fc dimer"(Fcmut arm / Fcmut arm)
- "sIFNbeta-IgGFcmut"(Fcmut arm / INFbeta-Fcmut arm heterodimer)
- "IFN-beta dimer" (INFbeta-Fcmut arm / INFbeta-Fcmut arm homodimer)
The purification sequence consists in enriching the "sIFNbeta-IgGFcmut" present in the supernatants while removing most of the related contaminants ("free Fc dimer", "IFN-beta dimer", truncated and aggregated forms) and non-related products (proteins from the medium and secreted by CHO cells). An initial capture step on Protein A binds all Fc-tagged molecules. The eluate is then applied onto a Blue Sepharose gel that preferably binds IFN-tagged molecules, and so, separates most of the "free Fc dimer".
The final two steps are an Ion Exchange Chromatography followed by a Size Exclusion Chromatography that behave as polishing steps and remove a significant part of "IFN-beta dimer" and high molecular weight species.
The process was run twice, each time with 3L of cell culture supernatant as starting material, to produce the required amount of Compound 1.

### 2.1. Capture step on Protein A

After PBS equilibration of the column packed with 53 ml of Mab Select Sure (GE Healthcare), the sample was loaded overnight onto the column by reducing the linear flow rate down to 35 cm/h. Then, the column was washed with PBS until getting the output signal back to the baseline. The elution was done by a single step at 100% of 0.1 M Glycine pH 2.7. The eluted peak was pooled and quantified by UV using a molar extinction coefficient of 100230 which gives A_{[280]} = 1.38 AU for a 1 mg/ml concentration.
The most abundant molecular species of the eluate (sIFNbeta-IgGFcmut, IFN-beta dimer and free Fc dimer) have similar extinction molecular coefficients, which allow a direct quantitation by UV spectrophotometry.
Then, the solution was diluted to 1.3 mg/ml in 0.1 M Glycine pH 2.7 and immediately neutralized by adding 20% (v/v) of 1 M Tris pH 8.

### 2.2. Purification on Blue Sepharose

After a buffer exchange step on G25, the protein content was quantified and the solution was applied onto Blue Sepharose 6 Fast Flow gel (GE Healthcare) in two parallel runs. sIFNbeta-IgGFcmut binds easily to the medium, whereas most of the free Fc dimer is recovered in the flowthrough fraction.
A first wash with Tris-HCl 50mM pH 8.5 removed residuals of free Fc dimer species, and a first elution step with 56% Tris-HCl 50mM pH 8.5 and 44% of a 45% propylene Glycol + 1.4 M NaCl + Tris-HCl 50 mM pH 8.5 solution removed some aggregates and IFN-beta dimers. The second elution step with 100% of the 45% propylene Glycol + 1.4 M NaCl + Tris-HCl 50 mM pH 8.5 buffer contained mainly the sIFNbeta-IgGFcmut and some IFN-beta dimers.

### 2.3. Polishing on Q Sepharose Fast Flow

The eluate from the Blue Sepharose step was buffer-exchanged in Tris 50mM pH 8.5, and injected on a Q Sepharose Fast Flow column. After a wash step with Tris 50mM pH 8.5, a salt gradient was applied reaching up to 0.5 M NaCl + Tris 50mM pH 8.5. The large and main peak containing the sIFNbeta-IgGFcmut was collected and quantified by UV-spectrophotometry.
This eluate was then applied onto a 10 kDa Centriprep filter unit (Amicon) to concentrate the material before the next and final step.

### 2.4. Polishing on Superdex 200

The protein solution was submitted to a size exclusion chromatography. In order to keep a high resolution, the injection volume was kept minimal, thus making multiple injections mandatory. As it was specified to formulate the protein in 50 mM Tris + 500 mM NaCl + 10% glycerol pH 7.5, the buffer used for the gel filtration was 50 mM Tris pH 7.5 + 500 mM NaCl. Only the central part of the sharp peak was collected in order not to contaminate the protein of interest with aggregates and IFN-beta dimers. Then, the protein was concentrated up to 1 mg/ml on Centriprep filters with a 10 kDa cut off and 10% glycerol was added. Finally, the solution was filtered on a 0.22 µm membrane and aliquoted. The vials are stored at - 80°C.
The final purified protein is made essentially of the siFNbeta-IgGFcmut. The free Fc dimer form is almost absent.
The purification protocol can deliver about 60 mg of purified Compound 1 per 3 liters of cell culture supernatants from the CHO protoclone.
SDS-PAGE analysis of the sIFNbeta-IgGFcmut has shown that both polypeptides of the protein are glycosylated (Fc domain and IFN-beta) and linked by disulfide bonds.
Compound 1 has thus shown a high degree of purity and activity. The final purified protein is made essentially of the sIFNbeta-IgGFcmut (the free Fc dimer form is almost absent).

### Example 3 - Productivity

Generation of a pool expressing Compound 1 was performed according to Examples 1 and 2. After transfection and selection, the expression of the molecule was tested, using a Biacore assay developed to measure IFN-β.
The pool was tested for secretion in 24 hrs pulse, using ProCH5 medium. A higher specific productivity was obtained at lower temperature.
Protoclone were isolated from a standard arraying at 1 cell/well. About 300 protoclones were selected and tested for productivity in HTS format.
The best 15 protoclones as tested in the HTS assay were tested in 24 hour pulse in 6 well and T75 flasks leading to selection of 3 candidate protoclones. These 3 protoclones were cloned by arraying at 0.4 cell/well. For each protoclone, 20 clones were selected; making sure that the plate from which they were selected met the statistical cloning criteria (< 33% of recovery, elimination of any wells with two colonies after visual inspection).
The clones were first screened in the HTS assay, and the best five for each originating from each protoclone were further tested in batch cultures in shake flasks. Initial seeding was at 0.2 x 10⁶ viable cells/ml of culture in ProCHO5 medium at 37°C. Shaking was at 130 rpm. The cultures were stopped at WD7 when viability started to decline. Cultures were tested for cell number, viability, titer, and an SDS-WB was performed to eliminate any clone showing abnormal molecule size and integrity. The two best clones showed titer up to 40 mg/lt and PCD around 4 at 37°C. Cell density was reasonable and growth was robust with no sign of early cell death, and intact molecule as detected in SDS-WB blot.
The two best clones were selected and retested further in shake-flask with temperature shift. The results did confirm the initial results obtained in shake flasks.
Titers up to 120 mg/lt, as detected by an IFN-β specific Biacore assay, were obtained by lowering temperature. This was obtained using a specific clone from which Compound 1 is derived. Robust growth and very good viability of the cells over the batch was observed. Specific productivity went up to 12 pcd. This clone thus showed an advantageous profile and very good potential for process development for Good Laboratory/Manufacturing Practice (GLP/GMP) material production. Viability and cell growth after thawing were verified according to standard protocol. Mycoplasma and sterility test were performed using FDA compliant protocols.

### Example 4 - Stability and Solubility

### 4.1 Batch used

Compound 1 diluted 1:2 in the original buffer: 50 mM Na Phosphate + 500 mM NaCl + 10% glycerol pH 7.5. Protein concentration in tests was 0.66 mg/ml.

### 4.2 Conditions tested

| | |
|---|---|
| thermal stability by SE-HPLC : | - 1 week at + 40°C |
| | - 1 month at + 5°C |
| | - 1 month at + 25°C |

freezing/thawing stability by SE-HPLC after 1 and 5 cycles (- 20°C)

### 4.3 Results

At time t=0 the sample has a sIFNbeta-IgGFcmut concentration of >98.5%, with less than 2% of HMWs.
After 1 week at + 40°C the sIFNbeta-IgGFcmut concentration decreases to about 81%, with about 19% of HMWs.
After 1 month at + 5°C siFNbeta-IgGFcmut concentration is about 99%, with a slight decrease in the HMWs content.
After 1 month at + 25°C both the duplicate samples showed, after the incubation, presence of precipitate. By SEC analysis, the expected peak of siFNbeta-IgGFcmut was eluted at a longer retention time, and other lower MW peaks were present. Further analysis by SDS-PAGE and N-terminal sequencing (by PVDF membrane transfer after SDS-Gel Electrophoresis) have confirmed the hypothesis of hydrolysis close to the fusion site, and no trace of IFN-β sequence was found.
After both 1 and 5 freeze/thaw cycles the sIFNbeta-IgGFcmut content is about 98%.

### 4.4 Freezing/Thawing Experiments

Samples are diluted to the testing concentrations and stored at + 4°C at least 24h for equilibration.
Then, after the SEC profile at t=0 is measured, samples are stored at - 20°C for at least one day, then thawed at + 4°C (for at least one day) and then analyzed by SEC.

### 4.5 Freezing/Thawing Experiments (5 cycles)

Samples are diluted down to the testing concentration and stored at +4°C at least 24h for equilibration.
Then, after the SEC profile at t=0 is measured, samples are stored at - 20°C for at least one day, then thawed at + 4°C (for at least one day), then frozen/thawed again up to five cycles with the same procedure.
At the end, after the fifth thawing cycle (and re-equilibration at 4°C for at least 24h), samples are analyzed by SEC.

### 4.6 Solubility

The protein was stored at - 80°C in the following buffer (preformulation):
50 mM Na Phosphate + 500 mM NaCl + 10% glycerol pH 7.5
The said batch of Compound 1 had the following concentration: 1.32 mg/ml.
Stability and F/T tests were performed on this batch in storage buffer.

### Example 5 - Biological behaviour of two IFN-β-Fc heterodimeric proteins: Compound 1 and Compound 2

### 5.1 Introduction

The biological behavior of two preparations of IFN-β-Fc heterodimeric proteins, Compound 1 and Compound 2 compared to recombinant human IFN-β1a (reference preparation 3 MIU/mL, 11 µg/mL) was verified in term of antiviral (WISH/VSV, Figure 5) and antiproliferative (WISH cells, Figure 6) activity as well as in a reporter gene assay (monkey Vero cells transfected with pMx-Luc, Figure 7).

### 5.2 Methods

### 5.2.1 Antiviral assay

The antiviral activity of IFN-β-Fc heterodimeric proteins was studied by measuring the protection exerted by the IFN-β molecule on WISH cells (human amnion cell line), against the cytopathic effect (CPE) of the vesicular stomatitis virus (VSV; Rubinstein et *al*. 1981).
WISH cells, grown in MEM + 10% FBS, were plated in MEM + 5% FBS at 4 × 10⁴ cells in each well of a 96-well microtiter containing IFN-β samples (starting concentration 0.7 ng/mL), at eight serial 1:1.5 fold dilution. Cells were then incubated for 18-22 hrs at 37°C, 5% CO₂. At the end of the incubation time, VSV suspension (in MEM + 2.5% FBS) was added and incubated for further 20-25 hrs at 37°C, 5% CO₂.
Cells were then stained with MTT (3-[4,5-Dimethylthiazol-2-yl]-2,5-Diphenyltetrazolium bromide) and the optical density (O.D. at 595 nm) in each well was measured by a multiwell scanning spectrophotometer.
The IFN-β-Fc preparations were tested in parallel in two independent experiments.
The dose-response curves thus generated were interpolated by a Sigmoidal dose-response (variable slope) curve (Graph Pad Software). The concentrations (pM) were reported on the x-axis and the relevant absorbance (OD) values, which were normalized vs. the control virus and the control cells (0 and 100% growth) were reported on the y-axis as percentage of inhibition of cytopatic effect.

### 5.2.2 Antiproliferative assay

WISH cells in MEM + 5% FBS were plated at 1 × 10⁴ in each well of a 96-well microtiter plate containing IFN-β samples (starting concentration 40 ng/ml), at nine serial 1:2 fold dilutions, then incubated for 72 hrs at 37°C, 5% CO₂. At the end of the incubation period, cells were stained with MTT and the optical density (O.D. at 595 nm) in each well measured by a multiwell scanning spectrophotometer.
The IFN-β-Fc preparations were tested in parallel in two independent experiments.
The dose-response curves thus generated were interpolated by a Sigmoidal dose-response (variable slope) curve (Graph Pad Software). The concentrations (pM) were reported on the x-axis and the OD values, which were normalized vs. the control cells (no IFN-β added, 100% growth), were reported on the y-axis as percentage of cell growth.
A different cell/virus system (e.g. A549/EMCV) and a different cell line (Hs294T) can also be used for the measurement of antiviral and antiproliferative activities.

### 5.2.3 Reporter gene assay

The reporter gene assay (RGA) was developed as a highly specific and sensitive in *vitro* bioassay for monitoring IFN-β activity. Vero cells (African green monkey kidney cells from ATCC, CCL 81), defective in endogenous IFN-α and IFN-β, were stably transfected with the pMx-Luc plasmid carrying the Luciferase (Luc) gene under the control of the IFN-α/β inducible murine Mx1 promoter. A clone was selected on the basis of Luc inducibility by IFN-β (Canosi *et al.* 1996).
The assay is based on the measurement of light emission caused by the oxidation of the Luciferin by Luciferase.
The amount of Luciferase produced by the transfected monkey Vero cells is a function of the amount of IFN-β added and is automatically measured by a luminometer connected to software.
The Vero cells were plated (40,000 cells) in each well of a 96-well microtiter plates containing IFN-β samples at seven serial 1:2 fold dilution with a starting concentration of 234 pg/mL. After about 21 hrs of incubation (37°C in 5% CO₂), the assay plate was inverted to remove the content; the cell layer was washed once with PBS and cell lysis buffer was added to each well. The plate was left about 15 min at room temperature and then introduced into the Victor Light 1420, where the Luciferase Assay Reagent (LAR) was automatically injected into each well and the light emitted, caused by the oxidation of the Luciferin by Luciferase, measured as count per second (cps).
The IFN-β-Fc preparations were tested in parallel in two experiments. The dose-response curves thus generated in each assay were interpolated by a Sigmoidal dose-response (variable slope) curve (Graph Pad Software), in which the IFN-β activity, expressed as mass (pg/mL), was reported on the x-axis and the relevant cps values, which were normalized vs. the basal value and the maximum value (0 and 100%), were reported on the y-axis as percentage of cps.
To calculate the actual IFN-β concentration to be added to the cells, the IFN-β mass contribution in the IFN-β-Fc was considered:
- Compound 1 : 0.94 mg/mL IFN-β-Fc = 0.26 mg/mL IFN-β
- Compound 2 : 1.10 mg/mL IFN-β-Fc = 0.31 mg/mL IFN-β

### 5.2.4 Results and conclusion

Regardless of the cell system used, the sIFNbeta-IgGFcmut (Compound 1) showed a bioactivity comparable to a monomer-dimer protein containing the single mutation N→A at position 297 according to EU index (Compound 2; Figures 5 to 7) and comparable to the recombinant human IFN-β preparation.

### Example 6 - Bioassay

The titre of both Compound 1 and Compound 2 was calculated on a theoretical basis and determined using the standard method (VSV-WISH) currently applied for the titre determination of IFN-β. Results are summarized in Table 2:

**Table 2**

| | Theoretical activity (MIU/mL) | Titre (MIU/mL) |
|---|---|---|
| Compound 1 | 100 | 81.8 |
| Compound 2 | 83 | 62.5 |

The theoretical activity is based on the following consideration:
For IFN-β (22kD), the conversion factor from MIU to [µg] is 3.66. So, 1 MIU corresponds to 3.66 µg.
For IFN-β-Fc (77 kD), if we assume that the mass specific activity is linear, the factor above is multiplied by 3.5 and ends to be 12.8. So, 1 MIU corresponds to 12.8 µg.

### Example 7 - Fc receptor/complement binding assays

### 7.1 Introduction

Because they contain antibody Fc portions, IFN-β fusion proteins have the potential to elicit effector functions associated with antibodies: antibody-dependent cell-mediated cytotoxicity (ADCC) and complement-dependent cytotoxicity (CDC).
In ADCC antigen-specific antibodies (e.g. IgG) direct immune effector cells of the innate immune system that express Fc gamma receptors (FcγRs) to the killing of antigen-expressing cells. FcγRs are expressed on NK cells, neutrophils, monocytes/macrophages, dendritic cells and B cells and comprise three classes: FcγRI (CD64), FcγRII (CD32) and FcγRIII (CD16). In human, the latter two classes are further divided into FcγRIIa and FcγRIIb, FcγRIIIa and FcγRIIIb. These receptors differ in their affinity for the various IgG subclasses and can be divided into high, intermediate or low affinity receptors. The high affinity receptor (CD64) binds monomeric IgG and is normally occupied by IgG in serum. The intermediate and low affinity receptors (CD16 and CD32, respectively) bind aggregated IgG and IgG-containing immune complexes. NK cells are believed to be the principle players in ADCC, although monocyte-macrophages and neutrophils may also play a role, and CD16 expressed by NK cells is considered as the 'ADCC receptor'. NK cells kill their target cells via the release of cytotoxic granules (perforin, granulysin and granzyme).

Complement activation via the so-called 'classical pathway' occurs upon binding of C1q to the Fc domain of immunoglobulins, IgG or IgM, complexed with antigens. C1q is a large complex glycoprotein of ∼410 kDa present in human serum at a concentration of around 70 µg/ml.

Together with two serine proteases, C1r and C1s, C1q forms the complex C1, the first component of complement. The capacity of the four human IgG subclasses (in monomeric form) to bind C1q decreases in the following order: IgG3>IgG1>IgG2>IgG4 (IgG4 does not activate complement). Hinge-dependent Fab-Fab and Fab-Fc flexibility determines the accessibility of the complement binding site to C1q, hence IgG3, the human IgG subclass with the longest hinge, is the most effective subclass in activating complement. The inability of IgG4 to activate complement is due to the structure of the Fc fragment as well as to the steric hindrance of the complement binding sites by the Fab arms.

Compound 1 contains modifications that reduce "effector functions", i.e. binding to Fcγ receptors and to C1q (Figures 1 and 2). To formally demonstrate the efficacy of these mutations, a study was carried out to compare the capacity of Compound 1 and Compound 2 to bind to one of the Fcγ receptors, FcγRI (CD64) and to C1q. Cell-based flow cytometry and ELISA-binding methods were employed.

### 7.2 Results

Compound 1 and Compound 2 show reduced binding to CD64 (FcγRI), compared to human IgG1.
Both IgG4 and IgG1 bound to CD64 expressed at the surface of transfected-AII1.6 cells, in a dose-dependent manner, with IgG4 showing slightly less binding than IgG1. By contrast, Compound 2 showed substantially less binding than human IgG1 and Compound 1 showed even a further reduction in binding, comparable to that obtained with a negative control (Figure 8). None of the molecules bind to the non-transfected IIA1.6 cells.
To characterize the binding of C1q to Compound 1 and Compound 2 an ELISA-C1q binding assay was carried out. Results are presented in Figure 9 (Fig. 9a and Fig. 9b). IgG1 displayed high C1q binding activity while no binding was detected for Compound 1, or the negative controls (Neg and IgG4). Compound 2 displayed a very slight binding. IgG2 did not bind C1q under these experimental conditions.
To investigate further the C1q binding capacity of Compound 1 and Compound 2, a surface plasmon resonance sensor was used to measure the affinity of these molecules for C1q. IgG1 served as a positive control, while IgG4 was included as a negative control.
The IFN-β-Fc molecules, along with the controls were individually immobilized on a CM5 chip by cross-linking primary amine residues to the dextran surface. Real-time association and dissociation of C1q with the immobilized proteins were monitored in a Biacore 3000 biosensor. The binding of C1q to IgG1 was characterized by a sharp increase of response during the association phase of the sensorgram and a slow decrease in the dissociation phase. Compound 2 showed very little binding to C1q, whereas Compound 1 showed no binding.
Thus, the mutations contained in Compound 1 completely hinder the binding of Compound 1 to C1q (Figure 9). The above-described studies demonstrate that the mutations introduced into the Fc portion of Compound 1 not only effectively reduce the binding of this molecule to CD64 (FcγRI)-transfected cells, but also abrogate the binding of Compound 1 to C1q in contrast to Compound 2. Similar results were obtained with non-transfected U937 human monocytic cells that express endogenous CD64. In the case of the CD64-transfected cells, the binding was identical to that obtained with a negative control.
In summary, the data obtained demonstrates that Compound 1 does not bind C1q under the experimental conditions employed in this study, while Compound 2 retains a low binding.

### Example 8 - PK/PD study in monkey

A pharmacokinetic/pharmacodynamic (PK/PD) study was performed in monkeys, the only animal species nearly-equally responsive to human IFN-β.
Both Compound 1 and Compound 2 were analyzed in serum samples using an enzyme immunoassay for the analysis of IFN-β serum levels in monkeys (FujiRebio, modified and validated in monkey serum for analysis of IFN-β).
Compound 1 and Compound 2, instead of the IFN-β standard, were used for the construction of the calibration curves.

### 8.1 Introduction

Compound 1 contains several mutations aimed to reduce binding to Fcγ receptors and to C1q. To check if these mutations altered the PK/PD properties of Compound 1, an *in vivo* study was performed in monkeys, the only animal species responsive to IFN-β. The study consisted of 3 groups of 5 Cynomolgus monkeys each. The first group received Rebif^{®}, by subcutaneous administration, at the dose of 55 pMol/kg, as reference. The 2^{nd} group of monkeys received Compound 2 while the 3^{rd} group of monkeys received Compound 1, both by inhalation at the intended deposited dose onto the lungs of 138 pMol/kg. IFN-β, Compound 1 and Compound 2 were measured, as well as the Neopterin serum levels. PK and PD analysis were performed after subtraction of any existing pre-dose concentration, according to non-compartmental analysis.

### 8.2 Pharmacokinetic Results

The mean (SD) serum profile of the IFNβ serum levels after administrations of Rebif® by subcutaneous route at the dose of 55 pMol/kg, of Compound 1 serum levels after administrations of Compound 1 by inhalation route at the nominal deposited dose of 138 pMol/kg, and of Compound 2 serum levels after administrations of Compound 2 by inhalation route at the nominal deposited dose of 138 pMol/kg are shown in Figure 10. After administration of Compound 1 or Compound 2 by inhalation, at the deposited (in the lungs) dose of 138 pMol/kg, the mean Cmax were 22.51 ± 7.43 pMol/L (Compound 2) and 16.36 ± 6.94 pMol/L (Compound 1) at a median time of 8 hours, to be compared with Rebif mean Cmax of 1.40 ± 1.10 pMol/L, reached at the median tmax of 3 hours. The AUClast was 886 ± 393 h.pMol/L for Compound 2 and 505 ± 322 h.pMol/L for Compound 1, to be compared with Rebif mean AUClast of 12.5 ± 20.0 h.pMol/L.
The apparent elimination half-life was 27 ± 4.5 h for Compound 2 and 24.1 ± 8.4 h for Compound 1. Both Compound 1 and Compound 2 showed regular and consistent serum levels, much higher than those observed with IFN-β. In fact both showed much higher cumulative exposure than that observed with injected Rebif^{®}.
The mean (SD) serum profile of baseline corrected Neopterin serum levels were calculated after administrations of Rebif^{®} by subcutaneous route at the dose of 55 pMol/kg, of Compound 1 by inhalation route at the nominal deposited dose of 138 pMol/kg, and of Compound 2 by inhalation route at the nominal deposited dose of 138 pMol/kg. The main pharmacodynamic parameters are shown in Table 3.

**Table 3: Main Pharmacodynamic parameters**

| | Rebif | Compound 2 | Compound 1 |
|---|---|---|---|
| Emax (nq/mL) | 3.87 (2.41) | 3.59 (1.03) | 2.86 (1.91) |
| AUElast (h.nq/mL) | 407 (270) | 552 (167) | 375 (252) |

Pre-dose Neopterin level in animals ranged between 1.11 and 5.06 ng/mL, vs. a value generally around 1 ng/mL. This fact should be taken into account since higher pre-dose levels may reduce the possibility to observe a further Neopterin increase.After subcutaneous administration of Rebife^{®}, a Neopterin increase was observed, as expected, in 4 of the 5 monkeys. One monkey showed no Neopterin increase. This monkey had the highest pre-dose Neopterin level (4.4 ng/mL) and no measurable IFN-β. The combination of these two factors (high Neopterin and possibly lower administered dose) may explain the lack of response. This is supported by the consideration that another monkey had a clear Neopterin response, despite the fact that only one serum sample showed measurable IFN-β levels. For this monkey the pre-dose concentration was 2.0 ng/mL. After administration of Compound 1 or Compound 2 by inhalation, at the deposited (in the lungs) dose of 138 pMol/kg, most animals showed a small increase of Neopterin levels. This small increase is more evident in some animals while others, despite the clear IFN-β-Fc PK profile, had only few serum samples exceeding the baseline concentration. Overall, both Compound 1 and Compound 2 showed a similar PK and PD behavior.

The following Table 4 will clarify the identity of the sequences reported in the Sequence Listing and throughout the text:

**Table 4**

| SEQ ID NO: | Sequence description |
|---|---|
| 1 | Heavy constant region of human IgG1 |
| 2 | Fragment of the hinge region of human IgG1 |
| 3 | Fcmut arm (i.e. a mutated IgG Fc arm not linked to a IFN-beta protein) |
| 4 | Sequence encoding Fcmut arm |
| 5 | Fcmut arm with signal peptide of mouse Igk light chain |
| 6 | Sequence encoding Fcmut arm with signal peptide of mouse Igk light chain |
| 7 | Human mature IFN-beta |
| 8 | IFNbeta-Fcmut arm (i.e. a mutated IgG Fc arm linked to a single IFN-beta protein) |
| 9 | Sequence encoding IFNbeta-Fcmut arm |
| 10 | IFNbeta-Fcmut arm with signal peptide of human IFN-beta |
| 11 | Sequence encoding IFNbeta-Fcmut arm with signal peptide of human IFN-beta |
| 12 | C370 vector |
| 13 | Linker of 8 amino acids |
| 14 | Subunit of Compound 2 not linked to IFN-beta |
| 15 | Subunit of Compound 2 linked to IFN-beta through a linker of 8 aa. |

### Reference list

1. Canosi U., M. Mascia, L. Gazza, O. Serlupi-Crescenzi, S. Donini, F. Antonetti, G. Galli, J. Immunol. Methods, 199, 69-76 (1996).
2. Kabat, E. A., Wu, T. T., Perry, H. M., Gottesman, K. S., and Foeller, C. (1991), Sequences of Proteins of Immunological Interest, 5th Ed., National Institutes of Health, Bethesda, MD.
3. Rubinstein, S.,Familletti, P.C., and Pestka, S. Convenient Assay for Interferons. J. Virol 1981; 37, 755-758.
4. Spiekermann GM, Finn PW, Ward ES, Dumont J, Dickinson BL, Blumberg RS, Lencer WI. Receptor-mediated immunoglobulin G transport across mucosal barriers in adult life: functional expression of FcRn in the mammalian lung. J Exp Med. 2002 Aug 5;196(3):303-10.
5. WO 2005/001025
6. US 6,485,726

## Claims

1. A protein comprising a single IFN-beta fused to a mutated IgG Fc domain containing two subunits, the first subunit comprises a mutated IgG Fc arm not linked to a IFN-beta protein, the second subunit comprises a mutated IgG Fc arm linked to a single IFN-beta protein, wherein said first subunit consists of SEQ ID NO: 3 and said second subunit consists of SEQ ID NO: 8.

2. A polynucleotide encoding a protein according to claim 1.

3. The polynucleotide according to claim 2, wherein said polynucleotide comprises or consists of SEQ ID NO: 4 or SEQ ID NO: 9.

4. The polynucleotide according to claim 2, wherein said polynucleotide comprises or consists of SEQ ID NO: 12.

5. An expression vector comprising a polynucleotide according to any of claims 2 to 4.

6. A host cell transformed with a vector according to claim 5.

7. The host cell according to claim 6, wherein said cell is a CHO cell.

8. A pharmaceutical composition comprising a protein according to claim 1.

9. A pharmaceutical composition according to claim 8 for use as a medicament.

10. Use of the pharmaceutical composition according to claim 9 for the preparation of a medicament for the treatment of multiple sclerosis.

11. A method of making a protein according to claim 1, wherein said method comprises culturing a host cell according to claim 6 or 7, and isolating said protein.

## Patentansprüche

1. Protein, umfassend ein einzelnes mit einer mutierten IgG-Fc-Domäne fusioniertes IFN-beta, das zwei Untereinheiten enthält, die erste Untereinheit umfasst einen mutierten 1gG-Fc-Arm, der nicht mit einem IFN-beta-Protein verknüpft ist, die zweite Untereinheit umfasst einen mutierten IgG-Fc-Arm, der mit einem einzelnen IFN-beta-Protein verknüpft ist, wobei die erste Untereinheit aus SEQ ID NO: 3 besteht und die zweite Untereinheit aus SEQ ID NO: 8 besteht.

2. Polynucleotid, das ein Protein gemäß Anspruch 1 kodiert.

3. Polynucleotid gemäß Anspruch 2, wobei das Polynucleotid SEQ ID NO: 4 oder SEQ ID NO: 9 umfasst oder daraus besteht.

4. Polynucleotid gemäß Anspruch 2, wobei das Polynucleotid SEQ ID NO: 12 umfasst oder daraus besteht.

5. Expressionsvektor, umfassend ein Polynucleotid gemäß einem der Ansprüche 2 bis 4.

6. Wirtszelle, die mit einem Vektor gemäß Anspruch 5 transformiert ist.

7. Wirtszelle gemäß Anspruch 6, wobei es sich bei der Zelle um eine CHO-Zelle handelt.

8. Pharmazeutische Zusammensetzung, umfassend ein Protein gemäß Anspruch 1.

9. Pharmazeutische Zusammensetzung gemäß Anspruch 8 zur Verwendung als Medikament.

10. Verwendung der pharmazeutischen Zusammensetzung gemäß Anspruch 9 zur Herstellung eines Medikaments für die Behandlung von multipler Sklerose.

11. Verfahren zur Herstellung eines Proteins gemäß Anspruch 1, wobei das Verfahren umfasst, eine Wirtszelle gemäß Anspruch 6 oder 7 zu züchten und das Protein zu isolieren.

## Revendications

1. Protéine comprenant une protéine d'IFN-β simple fusionnée à un domaine Fc d'IgG mutée comprenant deux sous-unités, une première sous-unité comprenant un bras de Fc d'IgG mutée qui n'est pas raccordé à une protéine d'IFN-β, et une deuxième sous-unité comprenant un bras de Fc d'IgG mutée raccordé à une protéine d'IFN-β simple, dans laquelle ladite première sous-unité consiste en la Séquence N° 3 et ladite deuxième sous-unité consiste en la Séquence N° 8.

2. Polynucléotide codant une protéine conforme à la revendication 1.

3. Polynucléotide conforme à la revendication 2, lequel polynucléotide comprend la Séquence N° 4 ou la Séquence N° 9, ou consiste en la Séquence N° 4 ou en la Séquence N° 9

4. Polynucléotide conforme à la revendication 2, lequel polynucléotide comprend la Séquence N° 12, ou consiste en la Séquence N° 12.

5. Vecteur d'expression comprenant un polynucléotide conforme à l'une des revendications 2 à 4.

6. Cellule hôte transformée avec un vecteur conforme à la revendication 5.

7. Cellule hôte conforme à la revendication 6, laquelle cellule est une cellule CHO.

8. Composition pharmaceutique comprenant une protéine conforme à la revendication 1.

9. Composition pharmaceutique conforme à la revendication 8, pour utilisation en tant que médicament.

10. Utilisation d'une composition pharmaceutique conforme à la revendication 9, en vue de la préparation d'un médicament destiné au traitement de la sclérose en plaques.

11. Procédé de préparation d'une protéine conforme à la revendication 1, lequel procédé comporte le fait de cultiver une cellule hôte conforme à la revendication 6 ou 7, et le fait d'isoler ladite protéine.


### SEQUENCE LISTING

<110> Laboratoires Serono SA
<120> Single IFN-beta fused to a mutated IgG Fc fragment
<130> 1162 WO/PCT
<150> EP 07118980.7
   <151> 2007-10-22
<150> US 61/007,142
   <151> 2007-12-11
<160> 15
<170> Patent In version 3.3
<210> 1
   <211> 330
   <212> PRT
   <213> homo sapiens
<220>
   <221> DOMAIN
   <222> (1)..(98)
   <223> CH1 domain
<220>
   <221> MISC_FEATURE
   <222> (99) . . (110)
   <223> Hinge region
<220>
   <221> DOMAIN
   <222> (111)..(223)
   <223> CH2 domain
<220>
   <221> DOMAIN
   <222> (229) . . (330)
   <223> CH3 domain
<400> 1
   Ala Ser Thr Lys Gly Pro Ser Val Phe Pro Leu Ala Pro Ser Ser Lys
   1               5                   10                  15
   Ser Thr Ser Gly Gly Thr Ala Ala Leu Gly Cys Leu Val Lys Asp Tyr
               20                  25                  30
   Phe Pro Glu Pro Val Thr Val Ser Trp Asn Ser Gly Ala Leu Thr Ser
           35                  40                  45
   Gly Val His Thr Phe Pro Ala Val Leu Gln Ser Ser Gly Leu Tyr Ser
       50                  55                  60
   Leu Ser Ser Val Val Thr Val Pro Ser Ser Ser Leu Gly Thr Gln Thr
   65                  70                  75                  80
   Tyr Ile Cys Asn Val Asn His Lys Pro Ser Asn Thr Lys Val Asp Lys
                   85                  90                  95
   Lys Val Glu Pro Lys Ser Cys Asp Lys Thr His Thr Cys Pro Pro Cys
               100                 105                 110
   Pro Ala Pro Glu Leu Leu Gly Gly Pro Ser Val Phe Leu Phe Pro Pro
           115                 120                 125
   Lys Pro Lys Asp Thr Leu Met Ile Ser Arg Thr Pro Glu Val Thr Cys
       130                 135                 140
   Val Val Val Asp Val Ser His Glu Asp Pro Glu Val Lys Phe Asn Trp
   145                 150                 155                 160
   Tyr Val Asp Gly Val Glu Val His Asn Ala Lys Thr Lys Pro Arg Glu
                   165                 170                 175
   Glu Gln Tyr Asn Ser Thr Tyr Arg Val Val Ser Val Leu Thr Val Leu
               180                 185                 190
   His Gln Asp Trp Leu Asn Gly Lys Glu Tyr Lys Cys Lys Val Ser Asn
           195                 200                 205
   Lys Ala Leu Pro Ala Pro Ile Glu Lys Thr Ile Ser Lys Ala Lys Gly
       210                 215                 220
   Gln Pro Arg Glu Pro Gln Val Tyr Thr Leu Pro Pro Ser Arg Glu Glu
   225                 230                 235                 240
   Met Thr Lys Asn Gln Val Ser Leu Thr Cys Leu Val Lys Gly Phe Tyr
                   245                 250                 255
   Pro Ser Asp Ile Ala Val Glu Trp Glu Ser Asn Gly Gln Pro Glu Asn
               260                 265                 270
   Asn Tyr Lys Thr Thr Pro Pro Val Leu Asp Ser Asp Gly Ser Phe Phe
           275                 280                 285
   Leu Tyr Ser Lys Leu Thr Val Asp Lys Ser Arg Trp Gln Gln Gly Asn
       290                 295                 300
   Val Phe Ser Cys Ser Val Met His Glu Ala Leu His Asn His Tyr Thr
   305                 310                 315                 320
   Gln Lys Ser Leu Ser Leu Ser Pro Gly Lys
                   325                 330
<210> 2
   <211> 7
   <212> PRT
   <213> Artificial sequence
<220>
   <223> Fragment of the hinge region
<400> 2
   Asp Lys Thr His Thr Cys Pro
   1               5
<210> 3
   <211> 227
   <212> PRT
   <213> Artificial sequence
<220>
   <223> Fcmut arm
<400> 3
   Asp Lys Thr His Thr Cys Pro Pro Cys Pro Ala Pro Glu Ala Glu Gly
   1               5                   10                  15
   Ala Pro Ser Val Phe Leu Phe Pro Pro Lys Pro Lys Asp Thr Leu Met
               20                  25                  30
   Ile Ser Arg Thr Pro Glu Val Thr Cys Val Val Val Asp Val Ser His
           35                  40                  45
   Glu Asp Pro Glu Val Lys Phe Asn Trp Tyr Val Asp Gly Val Glu Val
       50                  55                  60
   His Asn Ala Lys Thr Lys Pro Arg Glu Glu Gln Tyr Asn Ser Thr Tyr
   65                  70                  75                  80
   Arg Val Val Ser Val Leu Thr Val Leu His Gln Asp Trp Leu Asn Gly
                   85                  90                  95
   Lys Glu Tyr Lys Cys Lys Val Ser Asn Lys Ala Leu Pro Ser Ser Ile
               100                 105                 110
   Glu Lys Thr Ile Ser Lys Ala Lys Gly Gln Pro Arg Glu Pro Gln Val
           115                 120                 125
   Tyr Thr Leu Pro Pro Ser Arg Glu Glu Met Thr Lys Asn Gln Val Ser
       130                 135                 140
   Leu Thr Cys Leu Val Lys Gly Phe Tyr Pro Ser Asp Ile Ala Val Glu
   145                 150                 155                 160
   Trp Glu Ser Asn Gly Gln Pro Glu Asn Asn Tyr Lys Thr Thr Pro Pro
                   165                 170                 175
   Val Leu Asp Ser Asp Gly Ser Phe Phe Leu Tyr Ser Lys Leu Thr Val
               180                 185                 190
   Asp Lys Ser Arg Trp Gln Gln Gly Asn Val Phe Ser Cys Ser Val Met
           195                 200                 205
   His Glu Ala Leu His Asn His Tyr Thr Gln Lys Ser Leu Ser Leu Ser
       210                 215                 220
   Pro Gly Lys
   225
<210> 4
   <211> 681
   <212> DNA
   <213> Artificial sequence
<220>
   <223> Fcmut arm
<400> 4
   gacaaaactc acacatgccc accgtgccca gcacctgaag ccgagggggc accgtcagtc      60
   ttcctcttcc ccccaaaacc caaggacacc ctcatgatct cccggacccc tgaggtcaca     120
   tgcgtggtgg tggacgtgag ccacgaagac cctgaggtca agttcaactg gtacgtggac     180
   ggcgtggagg tgcataatgc caagacaaag ccgcgggagg agcagtacaa cagcacgtac     240
   cgtgtggtca gcgtcctcac cgtcctgcac caggactggc tgaatggcaa ggagtacaag     300
   tgcaaggtct ccaacaaagc cctcccatcc tccatcgaga aaaccatctc caaagccaaa     360
   gggcagcccc gagaaccaca ggtgtacacc ctgcccccat cccgggagga gatgaccaag     420
   aaccaggtca gcctgacctg cctggtcaaa ggcttctatc ccagcgacat cgccgtggag     480
   tgggagagca atgggcagcc ggagaacaac tacaagacca cgcctcccgt gctggactcc     540
   gacggctcct tcttcctcta cagcaagctc accgtggaca agagcaggtg gcagcagggg     600
   aacgtcttct catgctccgt gatgcatgag gctctgcaca accactacac gcagaagagc     660
   ctctccctgt ctccgggtaa a                                               681
<210> 5
   <211> 247
   <212> PRT
   <213> Artificial sequence
<220>
   <223> Fcmut arm with signal peptide of mouse Igk light chain
<220>
   <221> SIGNAL
   <222> (1)..(20)
   <223> Signal peptide of mouse Igk light chain
<220>
   <221> mast_peptide
   <222> (21) . . (247)
   <223> Fcmut arm
<400> 5
   Met Glu Thr Asp Thr Leu Leu Leu Trp Val Leu Leu Leu Trp Val Pro
   -20                 -15                 -10                 -5
   Gly Ser Thr Gly Asp Lys Thr His Thr Cys Pro Pro Cys Pro Ala Pro
               -1  1               5                   10
   Glu Ala Glu Gly Ala Pro Ser Val Phe Leu Phe Pro Pro Lys Pro Lys
           15                  20                  25
   Asp Thr Leu Met Ile Ser Arg Thr Pro Glu Val Thr Cys Val Val Val
       30                  35                  40
   Asp Val Ser His Glu Asp Pro Glu Val Lys Phe Asn Trp Tyr Val Asp
   45                  50                  55                  60
   Gly Val Glu Val His Asn Ala Lys Thr Lys Pro Arg Glu Glu Gln Tyr
                   65                  70                  75
   Asn Ser Thr Tyr Arg Val Val Ser Val Leu Thr Val Leu His Gln Asp
               80                  85                  90
   Trp Leu Asn Gly Lys Glu Tyr Lys Cys Lys Val Ser Asn Lys Ala Leu
           95                  100                 105
   Pro Ser Ser Ile Glu Lys Thr Ile Ser Lys Ala Lys Gly Gln Pro Arg
       110                 115                 120
   Glu Pro Gln Val Tyr Thr Leu Pro Pro Ser Arg Glu Glu Met Thr Lys
   125 130 135 140
   Asn Gln Val Ser Leu Thr Cys Leu Val Lys Gly Phe Tyr Pro Ser Asp
   145 150 155
   Ile Ala Val Glu Trp Glu Ser Asn Gly Gln Pro Glu Asn Asn Tyr Lys
   160 165 170
   Thr Thr Pro Pro Val Leu Asp Ser Asp Gly Ser Phe Phe Leu Tyr Ser
   175 180 185
   Lys Leu Thr Val Asp Lys Ser Arg Trp Gln Gln Gly Asn Val Phe Ser
   190 195 200
   Cys Ser Val Met His Glu Ala Leu His Asn His Tyr Thr Gln Lys Ser
   205 210 215 220
   Leu Ser Leu Ser Pro Gly Lys
   225
<210> 6
   <211> 741
   <212> DNA
   <213> Artificial
<220>
   <223> Fcmut arm with signal peptide of mouse Igk light chain
<220>
   <221> sig_peptide
   <222> (1)..(60)
   <223> Signal peptide of mouse Igk light chain
<220>
   <221> misc_feature
   <222> (61) . . (741)
   <223> Fcmut arm
<400> 6
   atggagacag acacactcct gctatgggta ctgctgctct gggttccagg ttccactggt 60
   gacaaaactc acacatgccc accgtgccca gcacctgaag ccgagggggc accgtcagtc 120
   ttcctcttcc ccccaaaacc caaggacacc ctcatgatct cccggacccc tgaggtcaca 180
   tgcgtggtgg tggacgtgag ccacgaagac cctgaggtca agttcaactg gtacgtggac 240
   ggcgtggagg tgcataatgc caagacaaag ccgcgggagg agcagtacaa cagcacgtac 300
   cgtgtggtca gcgtcctcac cgtcctgcac caggactggc tgaatggcaa ggagtacaag 360
   tgcaaggtct ccaacaaagc cctcccatcc tccatcgaga aaaccatctc caaagccaaa 420
   gggcagcccc gagaaccaca ggtgtacacc ctgcccccat cccgggagga gatgaccaag 480
   aaccaggtca gcctgacctg cctggtcaaa ggcttctatc ccagcgacat cgccgtggag 540
   tgggagagca atgggcagcc ggagaacaac tacaagacca cgcctcccgt gctggactcc 600
   gacggctcct tcttcctcta cagcaagctc accgtggaca agagcaggtg gcagcagggg 660
   aacgtcttct catgctccgt gatgcatgag gctctgcaca accactacac gcagaagagc 720
   ctctccctgt ctccgggtaa a 741
<210> 7
   <211> 166
   <212> PRT
   <213> homo sapiens
<400> 7
   Met Ser Tyr Asn Leu Leu Gly Phe Leu Gln Arg Ser Ser Asn Phe Gln
   1               5                   10                  15
   Cys Gln Lys Leu Leu Trp Gln Leu Asn Gly Arg Leu Glu Tyr Cys Leu
               20                  25                  30
   Lys Asp Arg Met Asn Phe Asp Ile Pro Glu Glu Ile Lys Gln Leu Gln
           35                  40                  45
   Gln Phe Gln Lys Glu Asp Ala Ala Leu Thr Ile Tyr Glu Met Leu Gln
       50                  55                  60
   Asn Ile Phe Ala Ile Phe Arg Gln Asp Ser Ser Ser Thr Gly Trp Asn
   65                  70                  75                  80
   Glu Thr Ile Val Glu Asn Leu Leu Ala Asn Val Tyr His Gln Ile Asn
                   85                  90                  95
   His Leu Lys Thr Val Leu Glu Glu Lys Leu Glu Lys Glu Asp Phe Thr
               100                 105                 110
   Arg Gly Lys Leu Met Ser Ser Leu His Leu Lys Arg Tyr Tyr Gly Arg
           115                 120                 125
   Ile Leu His Tyr Leu Lys Ala Lys Glu Tyr Ser His Cys Ala Trp Thr
       130                 135                 140
   Ile Val Arg Val Glu Ile Leu Arg Asn Phe Tyr Phe Ile Asn Arg Leu
   145                 150                 155                 160
   Thr Gly Tyr Leu Arg Asn
                   165
<210> 8
   <211> 393
   <212> PRT
   <213> Artificial sequence
<220>
   <223> IFNbeta-Fcmut arm
<220>
   <221> mat_peptide
   <222> (1)..(166)
   <223> Mature human IFN-beta
<220>
   <221> DOMAIN
   <222> (167) . . (393)
   <223> Fcmut arm
<400> 8
   Met Ser Tyr Asn Leu Leu Gly Phe Leu Gln Arg Ser Ser Asn Phe Gln
   1               5                   10                  15
   Cys Gln Lys Leu Leu Trp Gln Leu Asn Gly Arg Leu Glu Tyr Cys Leu
               20                  25                  30
   Lys Asp Arg Met Asn Phe Asp Ile Pro Glu Glu Ile Lys Gln Leu Gln
           35                  40                  45
   Gln Phe Gln Lys Glu Asp Ala Ala Leu Thr Ile Tyr Glu Met Leu Gln
       50                  55                  60
   Asn Ile Phe Ala Ile Phe Arg Gln Asp Ser Ser Ser Thr Gly Trp Asn
   65                  70                  75                  80
   Glu Thr Ile Val Glu Asn Leu Leu Ala Asn Val Tyr His Gln Ile Asn
                   85                  90                  95
   His Leu Lys Thr Val Leu Glu Glu Lys Leu Glu Lys Glu Asp Phe Thr
               100                 105                 110
   Arg Gly Lys Leu Met Ser Ser Leu His Leu Lys Arg Tyr Tyr Gly Arg
           115                 120                 125
   Ile Leu His Tyr Leu Lys Ala Lys Glu Tyr Ser His Cys Ala Trp Thr
       130                 135                 140
   Ile Val Arg Val Glu Ile Leu Arg Asn Phe Tyr Phe Ile Asn Arg Leu
   145                 150                 155                 160
   Thr Gly Tyr Leu Arg Asn Asp Lys Thr His Thr Cys Pro Pro Cys Pro
                   165                 170                 175
   Ala Pro Glu Ala Glu Gly Ala Pro Ser Val Phe Leu Phe Pro Pro Lys
               180                 185                 190
   Pro Lys Asp Thr Leu Met Ile Ser Arg Thr Pro Glu Val Thr Cys Val
           195                 200                 205
   Val Val Asp Val Ser His Glu Asp Pro Glu Val Lys Phe Asn Trp Tyr
       210                 215                 220
   Val Asp Gly Val Glu Val His Asn Ala Lys Thr Lys Pro Arg Glu Glu
   225                 230                 235                 240
   Gln Tyr Asn Ser Thr Tyr Arg Val Val Ser Val Leu Thr Val Leu His
                   245                 250                 255
   Gln Asp Trp Leu Asn Gly Lys Glu Tyr Lys Cys Lys Val Ser Asn Lys
               260                 265                 270
   Ala Leu Pro Ser Ser Ile Glu Lys Thr Ile Ser Lys Ala Lys Gly Gln
           275                 280                 285
   Pro Arg Glu Pro Gln Val Tyr Thr Leu Pro Pro Ser Arg Glu Glu Met
       290                 295                 300
   Thr Lys Asn Gln Val Ser Leu Thr Cys Leu Val Lys Gly Phe Tyr Pro
   305                 310                 315                 320
   Ser Asp Ile Ala Val Glu Trp Glu Ser Asn Gly Gln Pro Glu Asn Asn
                   325                 330                 335
   Tyr Lys Thr Thr Pro Pro Val Leu Asp Ser Asp Gly Ser Phe Phe Leu
               340                 345                 350
   Tyr Ser Lys Leu Thr Val Asp Lys Ser Arg Trp Gln Gln Gly Asn Val
           355                 360                 365
   Phe Ser Cys Ser Val Met His Glu Ala Leu His Asn His Tyr Thr Gln
       370                 375                 380
   Lys Ser Leu Ser Leu Ser Pro Gly Lys
   385 390
<210> 9
   <211> 1179
   <212> DNA
   <213> Artificial sequence
<220>
   <223> IFNbeta-Fcmut arm
<220>
   <221> misc_feature
   <222> (1) . . (498)
   <223> Mature human IFN-beta
<220>
   <221> misc_feature
   <222> (499) . . (1179)
   <223> Fcmut arm
<400> 9
   atgagctaca acttgcttgg attcctacaa agaagcagca attttcagtg tcagaagctc      60
   ctgtggcaat tgaatgggag gcttgaatat tgcctcaagg acaggatgaa ctttgacatc     120
   cctgaggaga ttaagcagct gcagcagttc cagaaggagg acgccgcatt gaccatctat     180
   gagatgctcc agaacatctt tgctattttc agacaagatt catctagcac tggctggaat     240
   gagactattg ttgagaacct cctggctaat gtctatcatc agataaacca tctgaagaca     300
   gtcctggaag aaaaactgga gaaagaagat ttcaccaggg gaaaactcat gagcagtctg     360
   cacctgaaaa gatattatgg gaggattctg cattacctga aggccaagga gtacagtcac     420
   tgtgcctgga ccatagtcag agtggaaatc ctaaggaact tttacttcat taacagactt     480
   acaggttacc tccgaaacga caaaactcac acatgcccac cgtgcccagc acctgaagcc     540
   gagggggcac cgtcagtctt cctcttcccc ccaaaaccca aggacaccct catgatctcc     600
   cggacccctg aggtcacatg cgtggtggtg gacgtgagcc acgaagaccc tgaggtcaag     660
   ttcaactggt acgtggacgg cgtggaggtg cataatgcca agacaaagcc gcgggaggag     720
   cagtacaaca gcacgtaccg tgtggtcagc gtcctcaccg tcctgcacca ggactggctg     780
   aatggcaagg agtacaagtg caaggtctcc aacaaagccc tcccatcctc catcgagaaa     840
   accatctcca aagccaaagg gcagccccga gaaccacagg tgtacaccct gcccccatcc     900
   cgggaggaga tgaccaagaa ccaggtcagc ctgacctgcc tggtcaaagg cttctatccc     960
   agcgacatcg ccgtggagtg ggagagcaat gggcagccgg agaacaacta caagaccacg    1020
   cctcccgtgc tggactccga cggctccttc ttcctctaca gcaagctcac cgtggacaag    1080
   agcaggtggc agcaggggaa cgtcttctca tgctccgtga tgcatgaggc tctgcacaac    1140
   cactacacgc agaagagcct ctccctgtct ccgggtaaa                           1179
<210> 10
   <211> 414
   <212> PRT
   <213> Artificial sequence
<220>
   <223> IFNbeta-Fcmut arm with signal peptide of human IFN-beta
<220>
   <221> SIGNAL
   <222> (1)..(21)
   <223> signal peptide of human IFN-beta
<220>
   <221> mat_peptide
   <222> (22) . . (187)
   <223> Mature human IFN-beta
<220>
   <221> DOMAIN
   <222> (188)..(414)
   <223> Fcmut arm
<400> 10
   Met Thr Asn Lys Cys Leu Leu Gln Ile Ala Leu Leu Leu Cys Phe Ser
       -20                 -15                 -10
   Thr Thr Ala Leu Ser Met Ser Tyr Asn Leu Leu Gly Phe Leu Gln Arg
   -5              -1  1               5                   10
   Ser Ser Asn Phe Gln Cys Gln Lys Leu Leu Trp Gln Leu Asn Gly Arg
               15                  20                  25
   Leu Glu Tyr Cys Leu Lys Asp Arg Met Asn Phe Asp Ile Pro Glu Glu
           30                  35                  40
   Ile Lys Gln Leu Gln Gln Phe Gln Lys Glu Asp Ala Ala Leu Thr Ile
       45                  50                  55
   Tyr Glu Met Leu Gln Asn Ile Phe Ala Ile Phe Arg Gln Asp Ser Ser
   60                  65                  70                  75
   Ser Thr Gly Trp Asn Glu Thr Ile Val Glu Asn Leu Leu Ala Asn Val
                   80                  85                  90
   Tyr His Gln Ile Asn His Leu Lys Thr Val Leu Glu Glu Lys Leu Glu
               95                  100                 105
   Lys Glu Asp Phe Thr Arg Gly Lys Leu Met Ser Ser Leu His Leu Lys
           110                 115                 120
   Arg Tyr Tyr Gly Arg Ile Leu His Tyr Leu Lys Ala Lys Glu Tyr Ser
       125                 130                 135
   His Cys Ala Trp Thr Ile Val Arg Val Glu Ile Leu Arg Asn Phe Tyr
   140                 145                 150                 155
   Phe Ile Asn Arg Leu Thr Gly Tyr Leu Arg Asn Asp Lys Thr His Thr
                   160                 165                 170
   Cys Pro Pro Cys Pro Ala Pro Glu Ala Glu Gly Ala Pro Ser Val Phe
               175                 180                 185
   Leu Phe Pro Pro Lys Pro Lys Asp Thr Leu Met Ile Ser Arg Thr Pro
           190                 195                 200
   Glu Val Thr Cys Val Val Val Asp Val Ser His Glu Asp Pro Glu Val
       205                 210                 215
   Lys Phe Asn Trp Tyr Val Asp Gly Val Glu Val His Asn Ala Lys Thr
   220                 225                 230                 235
   Lys Pro Arg Glu Glu Gln Tyr Asn Ser Thr Tyr Arg Val Val Ser Val
                   240                 245                 250
   Leu Thr Val Leu His Gln Asp Trp Leu Asn Gly Lys Glu Tyr Lys Cys
               255                 260                 265
   Lys Val Ser Asn Lys Ala Leu Pro Ser Ser Ile Glu Lys Thr Ile Ser
           270                 275                 280
   Lys Ala Lys Gly Gln Pro Arg Glu Pro Gln Val Tyr Thr Leu Pro Pro
       285                 290                 295
   Ser Arg Glu Glu Met Thr Lys Asn Gln Val Ser Leu Thr Cys Leu Val
   300                 305                 310                 315
   Lys Gly Phe Tyr Pro Ser Asp Ile Ala Val Glu Trp Glu Ser Asn Gly
                   320                 325                 330
   Gln Pro Glu Asn Asn Tyr Lys Thr Thr Pro Pro Val Leu Asp Ser Asp
               335                 340                 345
   Gly Ser Phe Phe Leu Tyr Ser Lys Leu Thr Val Asp Lys Ser Arg Trp
   350 355 360
   Gln Gln Gly Asn Val Phe Ser Cys Ser Val Met His Glu Ala Leu His
   365 370 375
   Asn His Tyr Thr Gln Lys Ser Leu Ser Leu Ser Pro Gly Lys
   380 385 390
<210> 11
   <211> 1242
   <212> DNA
   <213> Artificial sequence
<220>
   <223> IFNbeta-Fcmut arm with signal peptide of human IFN-beta
<220>
   <221> sig_peptide
   <222> (1)..(63)
   <223> Signal peptide of human IFN-beta
<220>
   <221> misc_feature
   <222> (64) . . (561)
   <223> Mature human IFN-beta
<220>
   <221> misc_feature
   <222> (562)..(1242)
   <223> Fcmut arm
<400> 11
   atgaccaaca agtgtctcct ccaaattgct ctcctgttgt gcttctccac tacagctctt      60
   tccatgagct acaacttgct tggattccta caaagaagca gcaattttca gtgtcagaag     120
   ctcctgtggc aattgaatgg gaggcttgaa tattgcctca aggacaggat gaactttgac     180
   atccctgagg agattaagca gctgcagcag ttccagaagg aggacgccgc attgaccatc     240
   tatgagatgc tccagaacat ctttgctatt ttcagacaag attcatctag cactggctgg     300
   aatgagacta ttgttgagaa cctcctggct aatgtctatc atcagataaa ccatctgaag     360
   acagtcctgg aagaaaaact ggagaaagaa gatttcacca ggggaaaact catgagcagt     420
   ctgcacctga aaagatatta tgggaggatt ctgcattacc tgaaggccaa ggagtacagt     480
   cactgtgcct ggaccatagt cagagtggaa atcctaagga acttttactt cattaacaga     540
   cttacaggtt acctccgaaa cgacaaaact cacacatgcc caccgtgccc agcacctgaa     600
   gccgaggggg caccgtcagt cttcctcttc cccccaaaac ccaaggacac cctcatgatc     660
   tcccggaccc ctgaggtcac atgcgtggtg gtggacgtga gccacgaaga ccctgaggtc     720
   aagttcaact ggtacgtgga cggcgtggag gtgcataatg ccaagacaaa gccgcgggag     780
   gagcagtaca acagcacgta ccgtgtggtc agcgtcctca ccgtcctgca ccaggactgg     840
   ctgaatggca aggagtacaa gtgcaaggtc tccaacaaag ccctcccatc ctccatcgag     900
   aaaaccatct ccaaagccaa agggcagccc cgagaaccac aggtgtacac cctgccccca     960
   tcccgggagg agatgaccaa gaaccaggtc agcctgacct gcctggtcaa aggcttctat    1020
   cccagcgaca tcgccgtgga gtgggagagc aatgggcagc cggagaacaa ctacaagacc    1080
   acgcctcccg tgctggactc cgacggctcc ttcttcctct acagcaagct caccgtggac    1140
   aagagcaggt ggcagcaggg gaacgtcttc tcatgctccg tgatgcatga ggctctgcac    1200
   aaccactaca cgcagaagag cctctccctg tctccgggta aa                       1242
<210> 12
   <211> 11014
   <212> DNA
   <213> Artificial sequence
<220>
   <223> C370 vector
<220>
   <221> misc_feature
   <222> (2499)..(3182)
   <223> Fcmut arm (reverse complementary strand)
<220>
   <221> sig_peptide
   <222> (3183)..(3242)
   <223> Signal peptide of mouse Igk light chain (reverse complementary strand)
<220>
   <221> sig_peptide
   <222> (6548)..(6610)
   <223> Signal peptide of human IFN-beta
<220>
   <221> misc_feature
   <222> (6611)..(7108)
   <223> Mature human IFN-beta
<220>
   <221> misc_feature
   <222> (7109)..(7789)
   <223> Fcmut arm
<400> 12
<210> 13
   <211> 8
   <212> PRT
   <213> Artificial sequence
<220>
   <223> Linker
<400> 13
   Glu Phe Ala Gly Ala Ala Ala Val
   1               5
<210> 14
   <211> 227
   <212> PRT
   <213> Artificial sequence
<220>
   <223> Subunit of Compound 2 without IFN-beta
<400> 14
   Asp Lys Thr His Thr Cys Pro Pro Cys Pro Ala Pro Glu Leu Leu Gly
   1               5                   10                  15
   Gly Pro Ser Val Phe Leu Phe Pro Pro Lys Pro Lys Asp Thr Leu Met
               20                  25                  30
   Ile Ser Arg Thr Pro Glu Val Thr Cys Val Val Val Asp Val Ser His
           35                  40                  45
   Glu Asp Pro Glu Val Lys Phe Asn Trp Tyr Val Asp Gly Val Glu Val
       50                  55                  60
   His Asn Ala Lys Thr Lys Pro Arg Glu Glu Gln Tyr Ala Ser Thr Tyr
   65                  70                  75                  80
   Arg Val Val Ser Val Leu Thr Val Leu His Gln Asp Trp Leu Asn Gly
                   85                  90                  95
   Lys Glu Tyr Lys Cys Lys Val Ser Asn Lys Ala Leu Pro Ala Pro Ile
               100                 105                 110
   Glu Lys Thr Ile Ser Lys Ala Lys Gly Gln Pro Arg Glu Pro Gln Val
           115                 120                 125
   Tyr Thr Leu Pro Pro Ser Arg Asp Glu Leu Thr Lys Asn Gln Val Ser
       130                 135                 140
   Leu Thr Cys Leu Val Lys Gly Phe Tyr Pro Ser Asp Ile Ala Val Glu
   145                 150                 155                 160
   Trp Glu Ser Asn Gly Gln Pro Glu Asn Asn Tyr Lys Thr Thr Pro Pro
   165 170 175
   Val Leu Asp Ser Asp Gly Ser Phe Phe Leu Tyr Ser Lys Leu Thr Val
   180 185 190
   Asp Lys Ser Arg Trp Gln Gln Gly Asn Val Phe Ser Cys Ser Val Met
   195 200 205
   His Glu Ala Leu His Asn His Tyr Thr Gln Lys Ser Leu Ser Leu Ser
   210 215 220
   Pro Gly Lys
   225
<210> 15
   <211> 401
   <212> PRT
   <213> Artificial sequence
<220>
   <223> Subunit of Compound 2 with IFN-beta
<220>
   <221> mat_peptide
   <222> (1)..(166)
   <223> Mature human IFN-beta
<220>
   <221> PEPTIDE
   <222> (167)..(174)
   <223> Linker
<220>
   <221> DOMAIN
   <222> (175)..(401)
   <223> Fc portion
<400> 15
   Met Ser Tyr Asn Leu Leu Gly Phe Leu Gln Arg Ser Ser Asn Phe Gln
   1 5 10 15
   Cys Gln Lys Leu Leu Trp Gln Leu Asn Gly Arg Leu Glu Tyr Cys Leu
   20 25 30
   Lys Asp Arg Met Asn Phe Asp Ile Pro Glu Glu Ile Lys Gln Leu Gln
   35 40 45
   Gln Phe Gln Lys Glu Asp Ala Ala Leu Thr Ile Tyr Glu Met Leu Gln
   50 55 60
   Asn Ile Phe Ala Ile Phe Arg Gln Asp Ser Ser Ser Thr Gly Trp Asn
   65 70 75 80
   Glu Thr Ile Val Glu Asn Leu Leu Ala Asn Val Tyr His Gln Ile Asn
   85 90 95
   His Leu Lys Thr Val Leu Glu Glu Lys Leu Glu Lys Glu Asp Phe Thr
   100 105 110
   Arg Gly Lys Leu Met Ser Ser Leu His Leu Lys Arg Tyr Tyr Gly Arg
   115 120 125
   Ile Leu His Tyr Leu Lys Ala Lys Glu Tyr Ser His Cys Ala Trp Thr
   130 135 140
   Ile Val Arg Val Glu Ile Leu Arg Asn Phe Tyr Phe Ile Asn Arg Leu
   145 150 155 160
   Thr Gly Tyr Leu Arg Asn Glu Phe Ala Gly Ala Ala Ala Val Asp Lys
   165 170 175
   Thr His Thr Cys Pro Pro Cys Pro Ala Pro Glu Leu Leu Gly Gly Pro
   180 185 190
   Ser Val Phe Leu Phe Pro Pro Lys Pro Lys Asp Thr Leu Met Ile Ser
   195 200 205
   Arg Thr Pro Glu Val Thr Cys Val Val Val Asp Val Ser His Glu Asp
   210 215 220
   Pro Glu Val Lys Phe Asn Trp Tyr Val Asp Gly Val Glu Val His Asn
   225 230 235 240
   Ala Lys Thr Lys Pro Arg Glu Glu Gln Tyr Ala Ser Thr Tyr Arg Val
   245 250 255
   Val Ser Val Leu Thr Val Leu His Gln Asp Trp Leu Asn Gly Lys Glu
   260 265 270
   Tyr Lys Cys Lys Val Ser Asn Lys Ala Leu Pro Ala Pro Ile Glu Lys
   275 280 285
   Thr Ile Ser Lys Ala Lys Gly Gln Pro Arg Glu Pro Gln Val Tyr Thr
   290 295 300
   Leu Pro Pro Ser Arg Asp Glu Leu Thr Lys Asn Gln Val Ser Leu Thr
   305                 310                 315                 320
   Cys Leu Val Lys Gly Phe Tyr Pro Ser Asp Ile Ala Val Glu Trp Glu
                   325                 330                 335
   Ser Asn Gly Gln Pro Glu Asn Asn Tyr Lys Thr Thr Pro Pro Val Leu
               340                 345                 350
   Asp Ser Asp Gly Ser Phe Phe Leu Tyr Ser Lys Leu Thr Val Asp Lys
           355                 360                 365
   Ser Arg Trp Gln Gln Gly Asn Val Phe Ser Cys Ser Val Met His Glu
       370                 375                 380
   Ala Leu His Asn His Tyr Thr Gln Lys Ser Leu Ser Leu Ser Pro Gly
   385                 390                 395                 400
   Lys